(19) **Europäisches Patentamt / European Patent Office / Office européen des brevets**

(11) **EP 1 784 480 B1**

(12) **FASCICULE DE BREVET EUROPEEN**

(45) Date de publication et mention
de la délivrance du brevet:
**12.07.2017 Bulletin 2017/28**

(21) Numéro de dépôt: **05798517.8**

(22) Date de dépôt: **16.08.2005**

(51) Int Cl.:
***C12M 1/34*** *(2006.01)* ***G01N 27/22*** *(2006.01)*

(86) Numéro de dépôt international:
**PCT/FR2005/002091**

(87) Numéro de publication internationale:
**WO 2006/021691 (02.03.2006 Gazette 2006/09)**

(54) **PROCÉDÉ ET DISPOSITIF DE DÉTERMINATION DE BIOMASSE DANS UN MILIEU, NOTAMMENT D'UN MILIEU CONTENANT DES CELLULES BIOLOGIQUES, ET APPAREIL DE MESURE METTANT EN OEUVRE CE PROCÉDÉ**

VERFAHREN UND VORRICHTUNG ZUR BIOMASSENBESTIMMUNG IN EINEM MEDIUM, INSBESONDERE EINEM BIOLOGISCHE ZELLEN ENTHALTENDEN MEDIUM, SOWIE MESSVORRICHTUNG UNTER VERWENDUNG DES VERFAHRENS

METHOD AND DEVICE FOR BIOMASS DETERMINATION IN A MEDIUM, IN PARTICULAR A MEDIUM CONTAINING BIOLOGICAL CELLS, AND MEASUREMENT APPARATUS IMPLEMENTING SAID METHOD

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **16.08.2004 FR 0408907**

(43) Date de publication de la demande:
**16.05.2007 Bulletin 2007/20**

(73) Titulaire: **Hamilton Bonaduz AG
7402 Bonaduz (CH)**

(72) Inventeurs:
• **OSSART, Frédéric**
  **30980 Langlade (FR)**
• **GHOMMIDH, Charles**
  **34980 Saint Gely du Fesc (FR)**
• **ESTEBAN, Geoffrey**
  **F-30000 Nimes (FR)**

(74) Mandataire: **Trossin, Hans-Jürgen
Ruttensperger Lachnit Trossin Gomoll
Patent- und Rechtsanwälte PartG mbB
Arnulfstraße 58
DE-80335 München (DE)**

(56) Documents cités:
**EP-A- 1 085 316    EP-A- 1 138 758
US-A- 4 214 203    US-B1- 6 496 020**

• **BORDI F, CAMETTI C AND GILI T: "Reduction of the contribution of electrode polarization effects in the radiowave dielectric measurements of highly conductive biological cell suspensions" BIOELECTROCHEMISTRY, vol. 54, 31 août 2001 (2001-08-31), pages 53-61, XP002320839 cité dans la demande**
• **JOHN E YARDLEY, ROBERT TODD, DAVID J NICHOLSON, JOHN BARRETT, DOUGLAS B KELL, CHRISTOPHER L DAVEY: "Correction of the influence of baseline artefacts and electrode polarisation on dielectric spectra", BIOELECTROCHEMISTRY, vol. 51, 29 February 2000 (2000-02-29), pages 53-65,**

**Description**

La présente invention concerne un procédé de détermination de biomasse dans un milieu, notamment un milieu contenant des cellules biologiques. Elle vise également un dispositif pour la mise en oeuvre de ce procédé, ainsi qu'un appareil de mesure mettant en oeuvre ce procédé, pouvant être utilisé pour une mesure de biomasse.

Il s'agit d'un procédé de détermination de caractéristiques diélectriques, qui permet d'une part la correction des principales sources d'erreurs rencontrées dans les mesures d'impédance appliquées aux milieux conducteurs, d'autre part l'obtention des paramètres caractérisant la dispersion β des suspensions de cellules biologiques.

Etat de l'art

Les mesures d'impédance électrique, et en particulier de permittivité diélectrique, peuvent être utilisées pour obtenir de manière non-destructive des informations sur la composition ou la structure des milieux étudiés. La mesure de la concentration de biomasse dans les milieux de fermentation est une application exemplaire de cette technique. Cette mesure, devenue aujourd'hui pratiquement une mesure de routine, est issue d'un développement en deux phases.

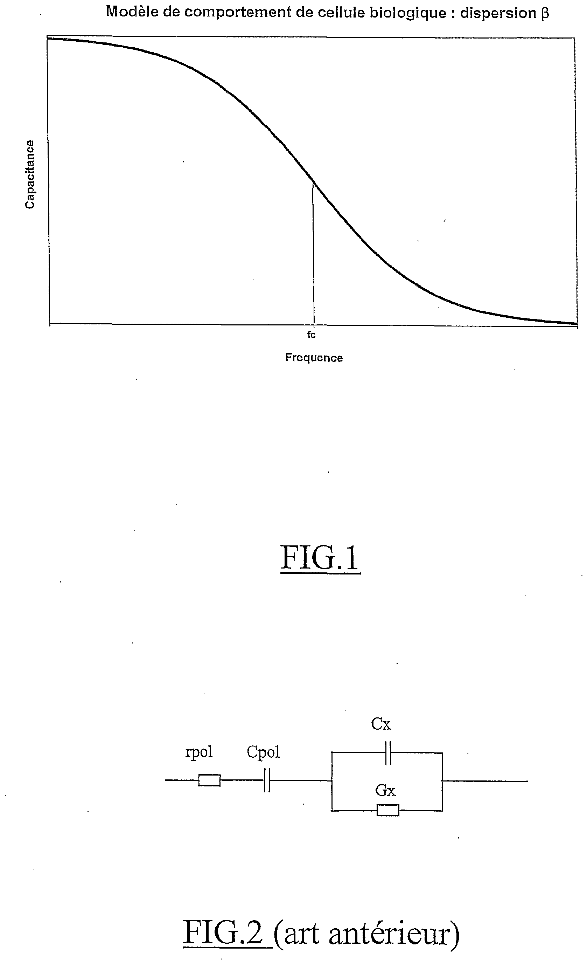

Une première phase, jusqu'à la fin des années 1970, a permis aux scientifiques d'étudier les propriétés électriques des milieux biologiques et des suspensions de cellules, et de montrer la relation entre concentration de cellules et permittivité diélectrique. On sait depuis les travaux de chercheurs tels que par exemple Fricke (1953, "Relation of the permittivity of biological cell suspensions to fractional cell volume" paru dans Nature, 172, 4381, 731-732) et Schwann, (1957, "Electrical properties of tissues and cell suspensions" paru dans Adv. Biol. Med. Phys., 5, 147-209), qu'il existe une corrélation forte entre permittivité, mesurée dans le domaine des fréquences radio et fraction volumique de cellules (levures, hématies...) dans le milieu étudié. En effet, lorsqu'une cellule vivante est soumise à un champ électrique, le déplacement des ions à l'intérieur de la cellule est limité par la membrane cytoplasmique, ce qui induit un phénomène de polarisation. Chaque cellule se comporte alors comme un petit condensateur. L'ampleur de cette polarisation, qu'on peut évaluer par mesure de la permittivité diélectrique (ou de la capacitance) du milieu, dépend de la fréquence du champ électrique appliqué. A fréquences relativement élevées, de l'ordre de 10 MHz et au delà, la polarisation est faible. La permittivité diélectrique (et la capacitance) correspond alors sensiblement à celle du milieu dépourvu de cellules. Au contraire, à fréquences relativement faibles, de l'ordre de 0.1 MHz, les cellules, sont complètement polarisées, et la permittivité diélectrique (et la capacitance de la suspension) est plus élevée. Ce phénomène, qui concerne donc les mesures réalisées dans le domaine des fréquences dites "radio", est décrit dans la littérature scientifique sous le nom de dispersion β. La forme de cette dispersion est caractéristique, comme l'illustre la figure 1, la permittivité diminuant progressivement depuis un plateau à fréquence basse jusqu'à un plateau à fréquence élevée, suivant une courbe en S inversé. La relation mathématique entre biovolume et permittivité diélectrique a été établie. Pour des cellules sphériques, l'incrément diélectrique $\Delta\varepsilon$, établit en faisant la différence entre la permittivité $\varepsilon_l$ mesurée à basse fréquence et celle $\varepsilon_h$ mesurée à fréquence plus élevée, est proportionnel au produit P.r.Cm, expression dans laquelle P est la fraction volumique occupée par la biomasse, r est le rayon des cellules, supposées sphériques, et Cm est la capacitance membranaire, selon la relation

$$\Delta\varepsilon = \frac{9\ p\ r\ C_m}{4\ \varepsilon_0}$$

Comme le volume cellulaire est à peu près proportionnel à la masse cellulaire, il est ainsi possible, grâce à deux mesures réalisées à deux fréquences de part et d'autre du domaine de la dispersion β, d'évaluer simplement la concentration de microorganismes (bactéries, levures, cellules animales...) dans un milieu de culture.

Siugura et al., dans l'article « Dielectric behavior of yeast cells in suspension », paru dans J.Gen.App.Microbiol., 10, 2, 163-174 (1964), ont ainsi présenté sans ambiguïté la relation expérimentale linéaire entre l'incrément diélectrique, mesuré dans un domaine de fréquence correspondant explicitement à la dispersion β, et la fraction volumique de suspensions de *Saccharomyces.*

Jusqu'à la fin des années 70, les travaux publiés ne concernaient le plus souvent que l'étude de suspensions "modèles" de cellules dans des milieux "idéaux", eau ou solutions salines à faibles concentrations. En effet, les mesures, qui utilisaient le plus souvent des ponts capacitifs à ajustement manuel et des électrodes en platine platiné, étaient fastidieuses et difficiles, empêchant ainsi tout développement pratique hors des laboratoires de recherche. Une des difficultés principales, dont nous reparlerons plus loin, rencontrées par les expérimentateurs était la polarisation de surface des électrodes, susceptible de gêner considérablement les mesures de capacitance. Malgré cela, Gencer et Mutharasan ont montré, dans l'article. "Determination of biomass concentration by capacitance measurement" paru en 1979 dans la revue Biotechnol. Bioeng., 21, 6, 1097-1103, l'intérêt des mesures de capacitance appliquées au suivi *in situ* et en temps réel des fermentations.

La deuxième phase commence à la fin des années 70, grâce aux progrès de l'électronique et au développement d'appareil automatisés, en particulier par la société Hewlett-Packard, qui changent alors radicalement la situation en simplifiant la mise en oeuvre des mesures de capacitance, permettant ainsi leur popularisation. (T. Ichino (HP) and H. Ohkawara (HP) and N. Sugihara (HP). Vector impedance analysis to 1000 MHz. Hewlett-Packard Journal: technical information from the laboratories of Hewlett-Packard Company, 31 (1), pp. 22-31, January 1980 ; Y. Narimatsu (HP) and K. Yagi (HP) and T. Shimizu (HP). A versatile low-frequency impedance analyzer with an integral tracking gain-phase meter. Hewlett-Packard Journal: technical information from the laboratories of Hewlett-Packard Company, 32 (9), pp. 22-28, September 1981).

Clarke et al., dans l'article « Sensors for bioreactor monitoring and control - a perspective, publié dans J. Biotechnol, 1, 135- 158 », sont parmi les premiers à citer explicitement l'intérêt de la technique pour la détermination de la concentration de biomasse en fermentation, et proposent un dispositif permettant, à partir d'une mesure de la permittivité, le suivi de la croissance de cultures microbiennes.

Le EP0282532 décrit une méthode de mesure de la biomasse qui permet, à partir d'une mesure de capacitance réalisée à une fréquence unique, choisie dans la domaine de fréquences basses de la dispersion β, d'obtenir un signal représentatif du biovolume. Cette méthode permet d'éviter les mesures à des fréquences multiples, nécessaires pour estimer l'amplitude de la dispersion β.

En revanche, ainsi qu'indiqué par l'inventeur lui-même (Yardley, Kell et al., 2000. On-line, real-time measurements of cellular biomass using dielectric spectroscopy, publié dans Biotechnology & Genetic Engineering Reviews, vol.17, 2000, Pages 3-35), il faut disposer d'une mesure de référence obtenue avant le début de la fermentation, pour pouvoir évaluer la variation de la capacitance. L'inconvénient principal de cette méthode est donc sa sensibilité aux erreurs liées aux variations des capacitances parasites, en fonction du temps, de la fréquence et de la conductivité du milieu, dues à la polarisation des électrodes ou à différentes imperfections des matériels utilisés.

Le EP0281602, déposé conjointement au brevet précédent, présente un appareil de mesure de la capacitance d'un milieu de fermentation, qui utilise une technique de mesure d'amplitude du signal, démodulé en quadrature et en phase;

Le EP0620919 décrit une méthode de mesure de la rétention gazeuse avec un appareil fonctionnant selon les principes décrit dans le brevet EP0281602, qui utilise deux mesures d'intensité du courant réactif à une fréquence élevée, l'une réalisée avant la fermentation, l'autre pendant la fermentation, le résultat étant utilisé pour corriger la mesure de biomasse;

Si le principe de la mesure est simple, sa mise en oeuvre est rendue complexe en raison de l'influence de plusieurs variables, qui agissent non seulement sur l'amplitude de l'incrément diélectrique, mais aussi sur la forme générale de la courbe de dispersion β. L'expression généralement retenue pour décrire cette dispersion est la suivante

$$\varepsilon = \varepsilon_h + \frac{\Delta\varepsilon\left[1 + \left(\frac{f}{f_c}\right)^{1-\alpha}\sin\alpha\frac{\pi}{2}\right]}{1 + \left(\frac{f}{f_c}\right)^{2(1-\alpha)} + 2\left(\frac{f}{f_c}\right)^{1-\alpha}\sin\alpha\frac{\pi}{2}}$$

Le point d'inflexion de cette courbe, situé à mi-hauteur entre les deux plateaux, correspond à une fréquence appelée fréquence caractéristique fc, comme l'illustre la figure 1. La pente de la tangente au point d'inflexion dépend de la superposition de plusieurs dispersions, de fréquences caractéristiques voisines, induites par exemple par une variation de la taille des cellules autour d'une valeur moyenne. Le coefficient α, qui permet de prendre ce phénomène en compte, est un paramètre empirique connu sous le nom de facteur de dispersion α de Cole-Cole.

L'article de Yardley et col. (précédemment cité) passe en revue l'ensemble des problèmes que pose la mesure. Par exemple, les contraintes techniques font que les mesures ne peuvent généralement être réalisées ni à fréquence suffisamment basse, ni à fréquence suffisamment élevée, ce qui fait que les plateaux de part et d'autre de la zone de dispersion β ne peuvent pas être atteints. On n'arrive donc pas à mesurer l'incrément diélectrique en totalité. La fréquence caractéristique fc se déplace sous l'influence des variations de la conductivité du milieu σm et de la conductivité intracellulaire σc, de la taille cellulaire r et de la capacitance membranaire Cm, selon la relation

$$f_c = \frac{1}{2\pi r\, C_m\left(\frac{1}{\sigma_c} + \frac{1}{2\sigma_m}\right)}$$

ce qui entraîne des variations de la mesure de capacitance si les mesures ne peuvent être réalisées sur les plateaux.

Enfin, la pente de la courbe de dispersion, autour de la fréquence caractéristique fc, est elle-même variable, en fonction de la valeur du facteur de dispersion α, ce qui peut induire des variations de la capacitance mesurée, indépendantes des variations de la concentration cellulaire.

Un article supplémentaire de Yardley qui est pertinent à l'invention actuelle est Yardley et al. Bioelectrochemistry 51 (2000) 53-65. Cet article considére les effets de la polarisation sur la spectroscopie diélectrique.

Il est ainsi souhaitable d'évaluer l'incrément diélectrique (ou capacitif) à partir de mesures réalisées à plusieurs fréquences, de manière à reconstituer, en extrapolant éventuellement, l'intégralité de la courbe de dispersion $\beta$. On parle alors de spectroscopie d'impédance. En raison de la forme de la fonction mathématique décrivant la dispersion $\beta$, l'obtention des descripteurs mathématiques de la courbe de dispersion ($\Delta\varepsilon$, fc, $\alpha$) demande généralement la mise en oeuvre de techniques mathématiques d'ajustement dites "non-linéaires", l'une des plus connues étant la méthode itérative de Levenberg-Marquard.

Ces techniques sont coûteuses en termes de puissance (ou de temps) de calcul. Par ailleurs, il est généralement nécessaire de fournir une valeur de départ aux différents paramètres pour que le processus itératif converge vers une solution convenable. Ces techniques ne peuvent donc pas être mises en oeuvre économiquement dans des systèmes de mesure à base de microcontrôleur, tels qu'on les rencontre dans de nombreux appareils de mesure commerciaux.

A ces difficultés s'ajoute celle liée à la polarisation de surface des électrodes. En effet, dans les systèmes de mesure d'impédance utilisant des électrodes en contact direct avec le milieu, la mesure des caractéristiques propres au milieu est perturbée en particulier par l'accumulation de charges à la surface des électrodes, qui provoque un phénomène de polarisation systématique. La capacitance qui en résulte vient s'ajouter à celle du milieu et évolue avec la conductivité ionique du milieu. Une deuxième source d'erreur est liée à l'adsorption de composés en solution sur les électrodes. Cette adsorption provoque une modification des propriétés électriques de l'interface métal-milieu liquide, qui se traduit, selon le type de molécules adsorbées, par une variation de la capacitance dont le sens et l'amplitude ne sont pas prévisibles, et que nous appellerons polarisation aléatoire. En pratique, l'amplitude de la polarisation aléatoire est nettement plus faible que celle de la polarisation systématique.

Ces problèmes sont bien connus, et plusieurs méthodes ont été proposées, en particulier par Schwan en 1963 dans son article "Determination of biological impedances", publié dans "Physical techniques in Biological research", vol 6, Nastuk ed., Academic Press, pp. 323-407, soit pour limiter l'ampleur de la polarisation (modification de l'état de surface des électrodes, systèmes à 4 électrodes, électrodes liquides), soit pour évaluer l'ampleur de la polarisation (variation de la distance inter-électrodes), soit pour évaluer et corriger par le calcul la contribution de la polarisation des électrodes. Au plan théorique, cette dernière opération est possible parce que la polarisation de surface des électrodes diminue rapidement avec la fréquence de mesure. En réalisant quelques mesures à basses fréquences, on peut évaluer la capacitance de polarisation aux fréquences supérieures, sous réserve que l'on connaisse sa loi de variation avec la fréquence. Une loi du type

$$C_{pol} = C_{0pol}.\ f^{-k}$$

est généralement utilisée, dans laquelle k est un coefficient expérimental généralement compris entre 1 et 2. On peut alors corriger les mesures de capacitance en leur soustrayant cette estimation de la capacitance de polarisation.

Ainsi, Sugiura et al. décrivent, dans l'article « Dielectric behavior of yeast cells in suspension », J.Gen.App.Microbiol., 10, 2, 163-174 (1964), une correction de la capacitance mesurée sur une suspension de levures, dans laquelle on utilise des mesures réalisées à basse fréquence pour corriger celles réalisées à fréquences plus élevées.

Le document EP 1 138 758 décrit, un procédé de mesure de la concentration et de la composition de biomasse par mesures d'impédance à plusieurs fréquences dans deux régions différentes, une région contenant des cellules et l'autre n'en contenant pas.

Le document EP 1 085 316 décrit un procédé de détection de changements de paramètres ou de conditions d'une suspension de cellules par des mesures d'impédance à plusieurs fréquences, lesdites mesures étant utilisées pour détecter des changements de la courbe d'impédance par rapport à une courbe de référence.

Bordi et al. ont proposé, dans l'article « Reduction of the contribution polarization effects in the radiowave dielectric measurements of highly conductive biological cell suspension », Biolectrochemistry 2001, une méthode globale d'ajustement non-linéaire qui permet l'élimination de la contribution de la capacitance des électrodes.

Le document EP0282532 (Kell) divulgue une méthode équivalente, dite méthode 2f, qui utilise le rapport des mesures de capacitance réalisées à deux fréquences dans la partie basse de la dispersion $\beta$, domaine de fréquence où l'influence de la polarisation des électrodes est prédominante par rapport à celle de la capacitance de la suspension cellulaire. L'inconvénient de cette dernière méthode est qu'elle suppose explicitement que la polarisation des électrodes suit une loi unique figée, indépendante de la conductivité du milieu, et surtout qu'elle ne prend pas en compte les erreurs dues aux imperfections des matériels utilisés.

Position du problème

A partir de 1993, la société Fogale a entrepris le développement d'un système de mesure avec pour but de résoudre les problèmes principaux posés par les mesures de capacitances en fermentation. L'objectif principal était le développement d'un système capable de réaliser les mesures de concentration de biomasse dans des milieux de conductivité électrique élevée, par exemple supérieure à 50 mS/cm, que l'on rencontre en conditions de fermentations industrielles. C'est le cas, lorsque les milieux de culture sont préparés à partir de mélasse de betterave, ou lorsque des acides organiques sont excrétés par les microorganismes cultivés comme lors des fermentations lactique, citrique ou gluconique. Un deuxième objectif était de parvenir à une géométrie de capteur permettant une utilisation non seulement en milieu industriel, mais également en laboratoire. Il fallait ainsi que la sonde de mesure ait un faible diamètre, pour s'adapter sur les ports standard de 12 mm, rencontrés sur tous les fermenteurs de petite taille, ce qui interdisait en pratique l'implantation de circuits actifs dans la sonde de mesure, au voisinage immédiat des électrodes, ainsi que pratiqué dans d'autres systèmes. Le brevet FR2812725 décrit ainsi un dispositif permettant d'atteindre ces objectifs, et de concevoir des sondes de mesure de grande longueur. Le brevet FR2835921 décrit une application à la mesure de biomasse de bactéries lactiques, en milieu fortement conducteur.

Plus spécifiquement, un appareil de mesure de biomasse tel que celui décrit dans le document FR2812725 présente des effets de ligne extrêmement élevés, qui viennent s'ajouter à la capacitance liée à la polarisation de surface des électrodes. L'erreur capacitive globale peut atteindre 150 pF/cm, ce qui est très largement supérieur aux valeurs correspondant spécifiquement à la biomasse présente dans les milieux de fermentation (typiquement 0.1 à 10 pF/cm).

Les méthodes de correction de polarisation en ligne citées précédemment ne tiennent pas compte de ces effets électroniques car les inventeurs de ces méthodes utilisaient des appareils conçus dans les règles de l'art, c'est à dire pourvus d'une électronique au plus près des électrodes (quelques mm) afin de minimiser les effets de lignes. Pour pouvoir utiliser un appareil de mesure de biomasse tel que celui décrit dans le document FR2812725, il est donc nécessaire de corriger non seulement les effets des polarisations systématique et aléatoire de la surface des électrodes, mais aussi les effets des autres sources d'erreur.

Le but de la présente invention est de proposer un procédé de détermination de biomasse dans un milieu, notamment un milieu comprenant des cellules en suspension dans un fluide, qui procure une correction efficace des erreurs observées dans les dispositifs de mesure existants.

Cet objectif est atteint avec un procédé d'évaluation d'une biomasse dans un milieu diélectrique à mesurer, l'évaluation de la biomasse étant obtenue à partir d'une différence entre un premier signal de capacitance dudit milieu, mesuré à une première fréquence, et un second signal de capacitance dudit milieu, mesuré à une seconde fréquence.

Suivant l'invention, le procédé comprend une correction séparée de chacun des signaux mesurés, selon au moins un niveau de correction, ce premier niveau de correction comprenant une correction desdits signaux mesurés selon un modèle dépendant de la conductance du milieu auxdites première et seconde fréquences.

Le procédé d'évaluation selon l'invention peut en outre avantageusement comprendre un second niveau de correction comprenant une correction desdits premier et second signaux de capacitance corrigés, à partir d'une troisième mesure de capacitance réalisée à une troisième fréquence, elle-même corrigée par une mesure de conductance réalisée à ladite troisième fréquence.

Lorsqu'il est mis en oeuvre pour un milieu comprenant des cellules en suspension dans un milieu, le procédé d'évaluation selon l'invention comprend en outre un troisième niveau de correction utilisant un modèle de comportement de la dispersion β dans ledit milieu.

Dans le procédé selon l'invention, les erreurs de capacitance dues à la polarisation aléatoire sont corrigées séparément de celles dues à la polarisation systématique et de celles dues à l'électronique, et il comprend en outre :

- une modélisation globale de la polarisation systématique et des erreurs de capacitance dues aux erreurs globales de l'électronique, sous la forme d'une équation commune $C_{cal}(G, f)$, fonction de la conductance du milieu et de la fréquence d'excitation des électrodes conductrices, de sorte que cette équation permet d'éliminer également le produit de l'effet combiné de la polarisation systématique et des erreurs globales de l'électronique au sein de l'appareil, et,

- une détermination d'une valeur de capacitance $Cm\_cor(G, f)$ corrigée, en comparant chaque mesure de capacitance brute $Cm(G, f)$ provenant de l'appareil et effectuée à une fréquence prédéterminée, à la valeur de ladite équation commune du modèle $C_{cal}(G, f)$ à ladite fréquence prédéterminée.

Ce procédé a été conçu pour être utilisé en ligne sur un appareil de mesure de biomasse tel que divulgué dans le document FR2812725 au nom du présent déposant, mais il peut cependant être appliqué à n'importe quel instrument de mesure d'impédance utilisant un capteur muni d'électrodes conductrices immergées dans un milieu diélectrique.

Le procédé selon l'invention corrige également les erreurs capacitives de l'électronique, les erreurs capacitives des effets de lignes du capteur, et les erreurs capacitives du capteur (les effets inductifs entre autres).

L'équation commune du modèle $C_{cal}(G, f)$ peut être de forme polynomiale, par exemple d'ordre 3 ou 4, et présenter des coefficients calculés pour une pluralité de fréquences prédéterminées utilisées par l'appareil, ou peut être approximée par un polynôme.

Les erreurs de capacitance corrigées par l'équation commune du modèle $C_{cal}(G, f)$ peuvent comprendre des erreurs capacitives fonction de la conductance et de la fréquence.

Les erreurs de capacitance corrigées par l'équation commune du modèle $C_{cal}(G, f)$ peuvent comprendre des erreurs liées à des effets de ligne.

Les erreurs de capacitance corrigées par l'équation commune du modèle $C_{cal}(G, f)$ peuvent comprendre des erreurs liées à des imperfections du capteur.

Le modèle de correction d'équation commune $C_{cal}(G, f)$ peut en outre être agencé pour éliminer l'incertitude sur la pente d'évolution de la polarisation systématique en fonction de la fréquence d'excitation.

Le modèle de correction d'équation commune $C_{cal}(G, f)$ peut en outre être agencé pour éliminer le produit de l'effet combiné de l'aération du milieu et des erreurs globales de l'électronique au sein de l'appareil.

Les coefficients du modèle de correction $C_{cal}(G, f)$ peuvent être déterminés à partir d'une opération de calibration dans un milieu de référence ne contenant pas de cellules biologiques et dont la conductance est modifiée de façon à parcourir la pleine échelle de la gamme de conductance de l'appareil.

La détermination de l'erreur de capacitance due à la polarisation aléatoire peut être effectuée à une fréquence prédéterminée $f_1$ choisie la plus basse possible de sorte que la détermination de l'erreur de capacitance de polarisation aléatoire soit peu influencée par la capacitance du milieu.

Lorsque le procédé selon l'invention est mis en oeuvre pour une mesure sur une suspension de cellules biologiques, la fréquence prédéterminée $f_1$ utilisée pour la détermination de l'erreur de capacitance due à la polarisation aléatoire peut être choisie inférieure ou égale à 100 kHz.

La capacitance de polarisation aléatoire $Cm\_cor(G, f_1)$ peut être calculée à la fréquence $f_1$ par différence entre la mesure de capacitance brute issue de l'appareil et le modèle de correction $Ccal(G, f_1)$.

Le procédé selon l'invention peut en outre comprendre une détermination de la capacitance des caractéristiques diélectriques du milieu à une deuxième fréquence prédéterminée $f_2$.

Lorsque le procédé selon l'invention est mis en oeuvre pour une mesure sur une suspension de cellules biologiques, la deuxième fréquence prédéterminée $f_2$ peut être choisie proche de la fréquence caractéristique fc de ladite suspension, caractéristique de la dispersion β des cellules en suspension.

La capacitance des caractéristiques diélectriques du milieu $Cm\_cor(G, f_2)$ peut être calculée à la fréquence $f_2$, par différence entre la mesure de capacitance brute issue de l'appareil et le modèle de conductance $Ccal(G, f_2)$.

Le procédé selon l'invention peut en outre comprendre une modélisation de la polarisation aléatoire selon un modèle de comportement tel que $a_{alea}. f^p. G^2$, dans lequel :

- G est la conductance du milieu,
- $a_{alea}$ est une constante prédéfinie,
- p est la pente de la polarisation.

La détermination de la capacitance du milieu diélectrique peut implémenter un modèle de correction résultant d'une combinaison :

- de la mesure de capacitance de la polarisation aléatoire $Cm\_cor(G, f_1)$, estimée à la première fréquence prédéterminée $f_1$,
- de la mesure de capacitance du milieu $Cm\_cor(G, f_2)$, estimée à la deuxième fréquence prédéterminée $f_2$,
- et du modèle de comportement de la polarisation aléatoire $a_{alea}. f^p. G^2$.

Lorsque le procédé selon l'invention est mis en oeuvre pour la mesure de suspensions de cellules biologiques suivant un modèle de comportement du type

$$\text{capacitance } Cx(G, f) = \Delta C_{cell} \times 1 / ( 1 + (f/f_c)^2 )$$

où $f_c$ est la fréquence caractéristique du milieu, cette fréquence caractéristique $f_c$ peut être soit prédéterminée à partir d'un abaque, soit déterminée en ligne par une méthode de détermination des paramètres caractéristiques de la dispersion β.

Le diélectrique mesuré à la première fréquence prédéterminée $f_1$ est de préférence sensiblement identique au diélectrique mesuré à la seconde fréquence prédéterminée $f_2$.

Dans une mise en oeuvre particulière du procédé selon l'invention pour la mesure d'un milieu contenant des cellules biologiques, ce procédé comprend en outre une détermination d'une erreur de capacitance Cm_cor(G, $f_3$) due à la dérive thermique de l'offset de l'électronique et à des variations de la capacitance du milieu diélectrique de suspension, à une troisième fréquence prédéterminée $f_3$.

L'erreur de capacitance Cm_cor(G, $f_3$) peut être calculée à la troisième fréquence prédéterminée $f_3$, par différence entre la mesure de capacitance brute issue de l'appareil et le modèle de correction Ccal(G, $f_3$).

Le procédé selon l'invention peut en outre comprendre une correction de mesures de capacitances brutes ou corrigées, entachées d'erreurs dues à la dérive thermique de l'offset de l'électronique et à des variations de capacitance du milieu de suspension, en soustrayant de ces mesures l'erreur de capacitance Cm_cor(G, $f_3$).

Il peut aussi comprendre une conversion des valeurs de capacitance et de conductance du milieu en des valeurs de permittivité et de conductivité, par multiplication desdites valeurs de capacitance et de conductance par un facteur de sonde k. qui est déterminé à partir d'une division de la valeur de conductivité d'une solution liquide de conductivité connue par une valeur de mesure de conductance de ladite solution.

Le procédé selon l'invention peut en outre comprendre une détermination d'un facteur de sonde $k_a$ lié à l'aération du milieu, à une quatrième fréquence prédéterminée $f_4$

La quatrième fréquence prédéterminée peut être choisie de sorte que le diélectrique est le plus stable quelque soient les changements de paramètres d'environnement.

Le facteur de sonde $k_a$ représente avantageusement une modification géométrique apparente du capteur lorsque des bulles sont présentes dans le milieu diélectrique.

Le procédé selon l'invention peut en outre comprendre une détermination de la capacitance Cm_cor(G, $f_4$) liée au facteur $k_a$ à la fréquence $f_4$, par comparaison de la mesure de capacitance brute au modèle de conductance Ccal(G, $f_4$). Le facteur de sonde $k_a$. est par exemple calculé en rapportant la variation de capacitance Cm_cor(G, $f_4$) à une valeur de capacitance d'un milieu de référence non aéré.

Le facteur de sonde $k_a$ peut être utilisé pour déterminer la permittivité du milieu diélectrique corrigée des effets d'aération sur la mesure de capacitance, et pour déterminer la conductivité du milieu en corrigeant les effets d'aération sur la mesure de conductance.

Le procédé selon l'invention peut en outre comprendre une détermination de la concentration de biomasse du milieu, en multipliant la permittivité mesurée à la seconde fréquence prédéterminée $f_2$ par un coefficient prédéterminé $\gamma$ qui peut être déterminé soit à l'aide d'un abaque de paramètres physiques caractéristiques des cellules biologiques, soit à l'aide d'un étalonnage préalable dans un milieu de suspension dont la concentration est connue.

On peut aussi avantageusement prévoir que le procédé selon l'invention, comprenne en outre une détermination de paramètres caractéristiques d'une dispersion diélectrique sur des milieux contenant des cellules biologiques, en utilisant au moins trois fréquences prédéterminées $f_5$, $f_6$, $f_7$, ainsi qu'une détermination de capacitances liées à la dispersion Cm_cor(G, $f_5$), Cm_cor(G, $f_6$), Cm_cor(G, $f_7$), aux au moins trois fréquences prédéterminée $f_5$, $f_6$, $f_7$, en comparant des mesures de capacitance brute provenant de l'appareil aux modèles de conductance aux fréquences correspondantes Ccal(G, $f_5$), Ccal(G, $f_6$), et Ccal(G, $f_7$).

La détermination des paramètres caractéristiques de la dispersion diélectrique peut comporter :

- une mesure d'un nombre n de valeurs de capacitance corrigée Cm_cor(G, $f_{5\ à\ m}$) avec m = 5 + n -1, à n fréquences réparties sur le domaine de fréquence correspondant à celui de la dispersion diélectrique étudiée, n étant supérieur ou égal à 3,
- un ajustement d'une fonction multilinéaire dépendant de la fréquence et comportant n coefficients variables pour approcher au mieux les n valeurs de capacitance corrigée mesurées,
- un calcul des paramètres caractéristiques de la dispersion diélectrique à partir des coefficients de la fonction multilinéaire.

La fonction multilinéaire dépendant de la fréquence peut être constituée par un polynôme de degré n-1. Le procédé selon l'invention peut en outre comprendre un calcul d'une évaluation de la concentration de biomasse du milieu, à partir des valeurs des coefficients du polynôme de degré n-1, ainsi qu'un calcul de l'évaluation de la taille des microorganismes dans le milieu, à partir des coefficients du polynôme de degré n-1.

Suivant un autre aspect de l'invention, il est proposé un dispositif pour évaluer une biomasse dans un milieu diélectrique à mesurer, mettant en oeuvre le procédé selon l'invention, comprenant un appareil générant un signal de capacitance et de conductance, cet appareil étant relié à un capteur ayant des électrodes conductrices agencées pour être en contact direct avec le milieu diélectrique à mesurer, et des moyens pour corriger le signal de capacitance, ce signal de capacitance étant entaché d'erreurs de capacitance dues à une polarisation aléatoire et d'erreurs de capacitance dues à une polarisation systématique.

Suivant l'invention, les moyens de correction du signal de capacitance sont agencés pour corriger séparément d'une part les erreurs de capacitance dues à la polarisation aléatoire et d'autre part les erreurs dues à la polarisation systématique, et comprennent :

- des moyens pour modéliser la polarisation systématique et des erreurs de capacitance incluant des erreurs dues à la polarisation systématique, sous la forme d'une équation commune $C_{cal}(G, f)$, fonction de la conductance du milieu et de la fréquence d'excitation des électrodes conductrices, cette équation étant agencée pour éliminer le produit de l'effet combiné de la polarisation systématique et des erreurs globales de l'électronique au sein de l'appareil, et,
- des moyens pour déterminer une valeur de capacitance $Cm\_cor(G, f)$ corrigée, en comparant chaque mesure de capacitance brute $Cx(G, f)$ provenant de l'appareil et effectuée à une fréquence prédéterminée, à la valeur de ladite équation commune du modèle $C_{cal}(G, f)$ à ladite fréquence prédéterminée.

Ce dispositif peut aussi en outre comprendre des moyens pour déterminer l'erreur de capacitance due à la polarisation aléatoire à une première fréquence prédéterminée $f_1$ choisie la plus basse possible de sorte que la détermination de l'erreur de capacitance de polarisation aléatoire est peu influencée par la capacitance du milieu., ainsi que des moyens pour déterminer la capacitance des caractéristiques diélectriques du milieu à une deuxième fréquence prédéterminée $f_2$ choisie proche de la fréquence caractéristique fc du milieu, caractéristique de la dispersion b des cellules en suspension.

Les moyens pour déterminer la capacitance des caractéristiques diélectriques du milieu peuvent implémenter un modèle de correction résultant d'une combinaison :

- d'une mesure de capacitance de la polarisation aléatoire $Cm\_cor(G, f_1)$, estimée à la première fréquence prédéterminée $f_1$,
- d'une mesure de capacitance du milieu $Cm\_cor(G, f_2)$, estimée à la deuxième fréquence prédéterminée $f_2$,
- et d'un modèle de comportement de la polarisation aléatoire $a_{alea} \cdot f^p \cdot G^2$ dans lequel G est la conductance du milieu, $a_{alea}$ est une constante prédéfinie, et p est la pente de la polarisation.

Le dispositif selon l'invention, mis en oeuvre pour la mesure d'un milieu contenant des cellules biologiques, peut en outre comprendre des moyens pour déterminer une erreur de capacitance $Cm\_cor(G, f_3)$ due à la dérive thermique de l'offset de l'électronique et à des variations de la capacitance du milieu diélectrique de suspension, à une troisième fréquence prédéterminée $f_3$, ainsi que des moyens pour déterminer une capacitance $Cm\_cor(G, f_4)$ liée à un facteur de sonde $k_a$ lié à l'aération du milieu, à une quatrième fréquence prédéterminée $f_4$.

Il peut aussi comprendre des moyens pour déterminer des paramètres caractéristiques d'une dispersion diélectrique sur des milieux contenant des cellules biologiques, en utilisant au moins trois fréquences prédéterminées $f_5, f_6, f_7$.

Dans une version particulière de l'invention, le dispositif comprend en outre des moyens pour déterminer des paramètres caractéristiques de la dispersion diélectrique, comportant :

- des moyens pour mesurer un nombre n de valeurs de capacitance corrigée $Cm\_cor(G, f_{5\ à\ m})$ à n fréquences réparties sur le domaine de fréquence correspondant à celui de la dispersion diélectrique étudiée, n étant supérieur ou égal à 3,
- des moyens pour ajuster une fonction multilinéaire dépendant de la fréquence et comportant n coefficients variables pour approcher au mieux les n valeurs de capacitance corrigée mesurées, et
- des moyens pour calculer des paramètres caractéristiques de la dispersion diélectrique à partir des coefficients de la fonction multilinéaire.

Suivant encore un autre aspect de l'invention, il est proposé un appareil pour évaluer une biomasse d'un milieu biologique, mettant en oeuvre un procédé d'évaluation selon l'invention, et/ou incluant un dispositif d'évaluation selon l'invention.

Il est aussi proposé dans le cadre de la présente invention un appareil de mesure d'impédance, agencé pour fournir une mesure de la partie réelle d'une impédance et de la capacitance correspondant à cette impédance, incluant un dispositif d'évaluation selon l'invention.

D'autres avantages et caractéristiques de l'invention apparaîtront à l'examen de la description détaillée d'un mode de mise en oeuvre nullement limitatif, et des dessins annexés sur lesquels :

- la figure 1 représente un modèle de comportement de cellules biologiques avec une dispersion $\beta$ ;
- la figure 2 illustre un modèle électrique conventionnel des effets de polarisation d'électrodes ;
- la figure 3 illustre une évolution d'une capacitance de polarisation mesurée sans ligne, en fonction de la fréquence ;
- les figures 4, 5 et 6 représentent, à différentes fréquences les erreurs capacitives respectivement dues aux imperfections de l'électronique, du capteur et aux effets de ligne ;

- la figure 7 représente sur un même graphe une courbe de polarisation absolue (polarisation systématique + polarisation aléatoire) et une courbe de polarisation systématique ;
- la figure 8 illustre un ajustement d'une fonction polynomiale d'ordre n sur une courbe de dispersion dans sa partie au-delà de la fréquence caractéristique, mis en oeuvre dans le procédé selon l'invention ; et

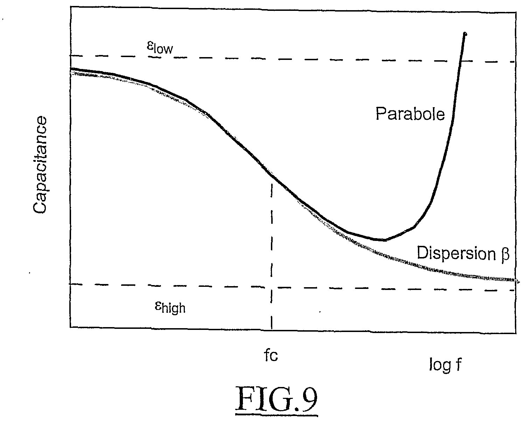

- la figure 9 illustre un ajustement d'une fonction polynomiale d'ordre n sur une courbe de dispersion dans sa partie en deçà de la fréquence caractéristique, mis en oeuvre dans le procédé selon l'invention.

Description de l'invention

On va tout d'abord décrire de façon détaillée les principes physiques à la base du procédé de détermination, en présentant une analyse des différentes sources d'erreur de capacitance rencontrées dans un appareil de mesure de biomasse du type de celui décrit dans le document FR2812725.

Le procédé selon l'invention propose une correction globale des effets parasites de l'électronique et des effets de polarisation d'électrode.

Les erreurs capacitives typiquement rencontrées dans un appareil de mesure de biomasse sont les suivantes :

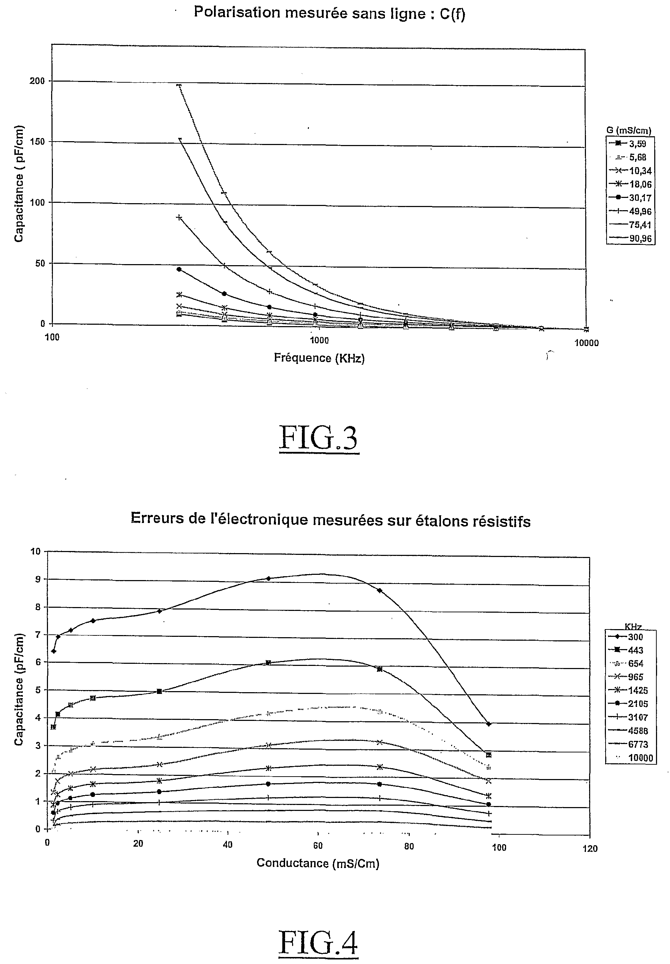

- des erreurs de l'électronique de mesure, telles qu'illustrées en figure 4. Ces erreurs, dont l'amplitude peut atteindre la dizaine de pF/cm, varient en fonction de la conductance G et de la fréquence. Elles sont générées par un nombre très important de composants, ce qui la rend difficile à corriger de façon théorique. Parmi les facteurs principaux intervenant dans ces erreurs, on peut citer les erreurs en phase des composants, les effets inductifs des pistes, et les erreurs non-linéaires des composants ;

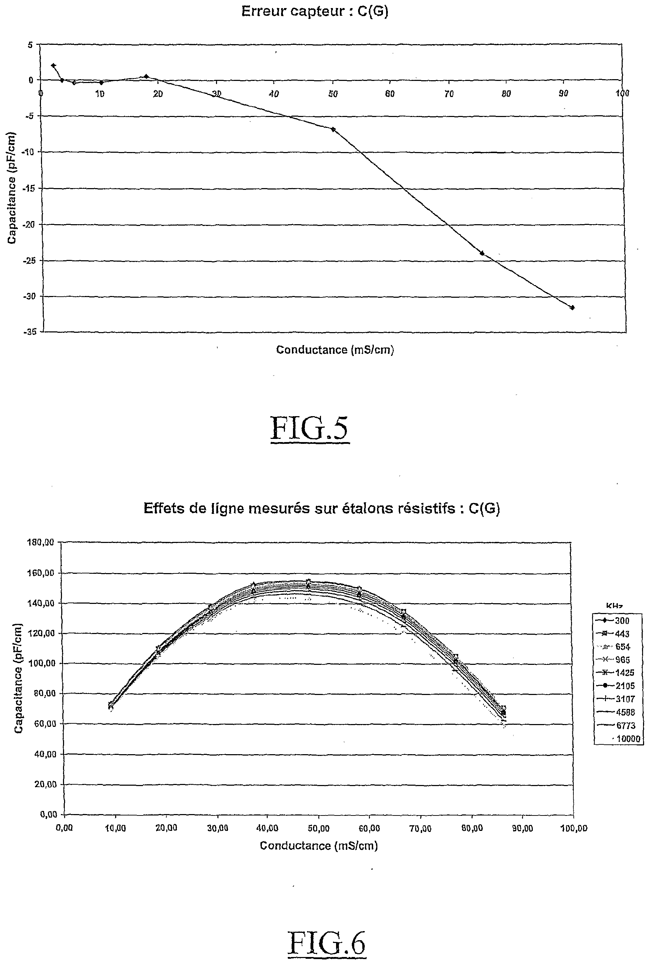

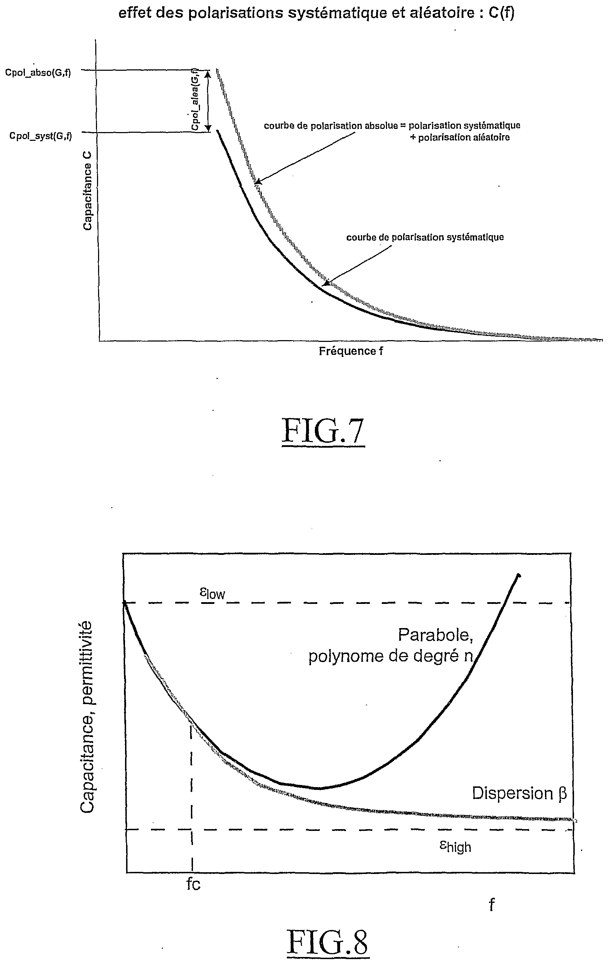

- des erreurs du capteur, telles qu'illustrées en figure 7. En dehors de l'effet de polarisation, les erreurs sur le capteur sont principalement de nature inductive, peuvent atteindre 30 pF/cm, et varient en fonction de la fréquence ;
- des erreurs d'effet de ligne, telles qu'illustrées en figure 6. En dehors de l'effet de polarisation, ce sont les erreurs les plus élevées rencontrées dans un appareil de mesure de biomasse du type de celui décrit dans le document FR2812725. Elles peuvent atteindre 150 pF/cm. Elles varient principalement en fonction de la fréquence et de la conductance G.

Le résultat de la mesure de permittivité dans un milieu diélectrique est la conséquence de l'ensemble des phénomènes électroniques présentés, auxquels s'ajoutent les phénomènes de polarisation de la surface des électrodes. Tous ces phénomènes ont chacun leur propre comportement en fonction de la conductance et de la fréquence et parfois ont un comportement non-linéaire comme dans le cas de l'électronique.

Il est donc nécessaire de développer une méthode pour extraire l'information "permittivité du milieu" du signal global de capacitance. Pour la mesure de biomasse, la difficulté d'extraction de la mesure "permittivité de la biomasse" est encore plus grande. Les cellules biologiques ont leur propre réponse fréquentielle, qui vient s'ajouter aux différentes réponses spectrales du matériel et de la polarisation du capteur.

Il existe déjà des méthodes de calcul en ligne, qui pour la plupart ne corrigent que les erreurs de polarisation. En effet, les systèmes de mesure de biomasse sont généralement dotés d'une électronique disposée au plus près des électrodes, conformément aux règles de l'art. Les effets de lignes sont alors beaucoup moins élevés que ceux observés dans un système où l'électronique se trouve déportée par rapport aux électrodes, et de ce fait, ne requièrent pas une méthode combinant correction de polarisation et de l'effet de ligne.

On connaît déjà une méthode de détermination de biomasse, développée par Bordi et al. (précité), qui utilise une formule générale qui est le résultat de la combinaison de modèles de fonctionnement physique, chimique, biologique et électronique. Cependant, il en résulte une utilisation très lourde car il est nécessaire de déterminer tous les coefficients provenant de ces modèles. De plus, une méthode de régression doit être mise en place afin de comparer la formule globale au spectre de mesure de capacitance. Le spectre doit comporter un nombre élevé de fréquences pour identifier tous les paramètres.

Le procédé selon l'invention permet de déterminer la permittivité d'un milieu diélectrique à partir d'un signal de capacitance et d'un signal de conductance, contenant les erreurs capacitives de l'électronique, des effets de lignes, du capteur et de la polarisation d'électrode.

De manière simplifiée, le procédé selon l'invention consiste à corriger les mesures de capacitance en utilisant les mesures de conductance réalisées simultanément par l'appareil. En effet, nous avons montré, à partir de considérations théoriques et de mesures expérimentales, que toutes les erreurs, qu'il s'agisse des erreurs de polarisation ou des erreurs liées aux imperfections de l'appareil, pouvaient être exprimée en fonction de la conductance du milieu. On corrige donc chaque mesure de capacitance en lui soustrayant une valeur de correction calculée à partir de la conductance, à l'aide d'une équation établie à l'issue d'un étalonnage préalable. Un deuxième niveau de correction permet ensuite d'éliminer les erreurs résiduelles pour conduire à une valeur de capacitance calculée qui ne dépend plus que des propriétés diélectriques du milieu étudié.

Ces signaux de mesures et les erreurs de l'électronique s'écrivent de la façon suivante :

$$Cm(G, f) = Cx(G, f) + Celec\_ampli(G, f) + Celect\_lin(G, f) + Celec\_sens(G, f) + Cpol\_syst(G, f) + Cpol\_al\acute{e}a\,(G, f) \quad (1)$$

avec :

- Cm(G, f) : signal de capacitance brute
- Cx(G, f) : capacitance propre du milieu diélectrique (biomasse)
- Celec_ampli(G, f) : capacitance de l'électronique de mesure
- Celect_lin(G, f) : capacitance de l'effet de ligne
- Celec_sens(G, f) : capacitance du capteur
- Cpol_syst(G, f) : capacitance de polarisation systématique
- Cpol_aléa(G, f) : capacitance de polarisation aléatoire.

On va maintenant décrire, en référence aux figures précitées, les principales étapes de réalisation du procédé de détermination selon l'invention.

Premier niveau de correction : élimination des erreurs liées à l'électronique et à la polarisation systématique

Etape 1 : modèle global de l'électronique

Le modèle global de l'électronique a été élaboré à la suite d'une étude sur les erreurs électroniques d'un appareil de mesure de biomasse, montrant que les courbes des erreurs capacitives (électronique, effets de ligne et erreurs liées au capteur) ont des formes en fonction de la conductance qui restent inchangées quel que soit le milieu. diélectrique mesuré. Par contre, ces courbes varient avec la fréquence utilisée.

Il en résulte que, pour s'affranchir des effets globaux de l'électronique et avant tout autre traitement, il convient de corriger le signal brut de capacitance, en lui soustrayant une valeur Celec_mod(G, f), calculée à partir d'un modèle de comportement global, fonction de la conductance (2), et ce, pour chacune des fréquences de mesure utilisées.

$$Celec\_mod(G, f) = Celec\_ampli(G, f) + Celect\_lin(G, f) + Celec\_sens(G, f) \quad (2)$$

Les erreurs capacitives du capteur et des effets de ligne sont principalement dues à des erreurs de type inductif (selfique). De façon théorique, on démontre que ce type d'erreur génère sur la mesure de capacitance une erreur proportionnelle à $-L.G^2$ (L : valeur de la self inductance, et G : valeur la conductance qui charge la self inductance). Ce modèle permet de corriger un pourcentage très élevé (typiquement 90%) des erreurs de l'électronique de l'appareil de mesure de biomasse. Afin de corriger l'erreur résiduelle et les erreurs de non-linéarité de l'électronique, on utilise en pratique une équation polynomiale d'ordre 3 ou 4, fonction de la conductance (3), calculée par la méthode des moindres carrés, selon une procédure qui sera décrite un peu plus loin. On pourrait envisager d'utiliser un polynôme d'ordre différent ou d'autres types de modèles, fonctions de la conductance. L'expression utilisée pour la première étape de correction est donc la suivante :

$$Celec\_mod(G, f) = e_{f,0} + e_{f,1} \cdot G + e_{f,2} \cdot G^2 + e_{f,3} \cdot G^3 \quad (3)$$

avec :

- $e_{f,i}$ : coefficients prédéterminés pour une fréquence donnée
- G : conductance du milieu

Etape 2 : modèle global corrigeant l'effet de polarisation systématique et les erreurs -globales de l'électronique

Cette seconde étape de correction utilise un modèle corrigeant la polarisation systématique séparément de la polarisation aléatoire. La polarisation absolue Cpol_abso(G, f) est la somme des deux polarisations, respectivement systématique et aléatoire, en référence à la figure 7.

$$Cpol\_abso(G, f) = Cpol\_syst(G, f) + Cpol\_alea(G, f) \qquad (4)$$

La polarisation systématique Cpol_syst(G, f) est la polarisation que l'on observe dans un bain de référence qui ne contient pas de cellules biologiques et dans lequel on fait varier la conductance en vue d'un étalonnage de l'appareil. Cette opération n'est effectuée qu'une seule fois.

La polarisation aléatoire Cpol_alea(G, f) est la modification de polarisation que l'on observe à un instant donné, par rapport à la polarisation systématique. Cette modification est imprévisible dans le temps.

Le modèle électrochimique de la polarisation systématique est le modèle connu de la double couche de Nernst. Son schéma électronique équivalent est présenté sur la figure 2, avec :

- rpol : résistance de polarisation
- Cpol : capacitance de la polarisation de la double couche
- Cx : capacitance du milieu
- Gx : conductance du milieu

En calculant l'effet de la capacitance de polarisation Cpol sur la mesure Cx à partir de ce modèle électrochimique, on trouve :

$$Cpol\_syst(G, f) = a_{sys}.\ f^p.\ G^2 \qquad (5)$$

avec :

- G : conductance du milieu
- $a_{sys}$ : constante prédéfinie (en général égale à 1/capacitance de polarisation systématique)
- p : constante correspondant à la pente de la polarisation (en général égale à -2)

Comme pour les erreurs d'électronique, on observe que pour une fréquence fixe, l'effet de polarisation ne varie qu'en fonction de la conductance. On a donc combiné les deux types d'erreurs, ce qui permet l'utilisation d'un modèle commun d'erreur de capacitance en fonction de G, qui regroupe l'erreur globale de l'électronique et l'erreur de polarisation systématique.

$$Ccal(G, f) = Celec\_mod(G, f) + Cpol\_syst(G, f) \qquad (6)$$

$$Ccal(G, f) = e_{f,0} + e_{f,1}.\ G + (a_{sys}.\ f^p + e_{f,2}).\ G^2 + e_{f,3}.\ G^3 \qquad (7)$$

on obtient alors un polynôme de la forme :

$$Ccal(G, f) = a_{f,0} + a_{f,1}.\ G + a_{f,2}.\ G^2 + a_{f,3}.\ G^3 \qquad (8)$$

avec:

- $a_{fi}$ : coefficients prédéterminés pour une fréquence donnée
- G : conductance du milieu

On pourrait envisager d'utiliser un polynôme d'ordre différent ou un autre type de modèle de conductance. Un modèle non-linéaire pourrait être utilisé si par exemple l'électronique ou la polarisation avaient un comportement non-linéaire en fonction de la conductance.

Cette méthode a l'avantage d'éliminer également les effets de second ordre comme le produit de l'effet combiné de la polarisation et des erreurs globales de l'électronique, comme l'incertitude sur la valeur de la pente (fixée à -2) de la polarisation systématique, et comme le produit de l'effet combiné de l'aération du milieu avec les erreurs globales de l'électronique.

Les coefficients du modèle (8) doivent être déterminés expérimentalement pour chaque fréquence de mesure utilisée par l'appareil de mesure de biomasse. Ceci est réalisé par une opération d'étalonnage dans un milieu de référence ne contenant pas de cellules biologiques et dans lequel on fait varier la conductance du milieu sur toute la gamme de l'appareil. Les valeurs de conductance et de capacitance obtenues permettent de calculer les coefficients du modèle (8).

Si la permittivité de ce milieu de référence est connue, alors elle peut être soustraite des mesures de capacitance avant le calcul des coefficients. Cela permet d'obtenir un modèle de correction, fonction de la conductance et exempt de la capacitance du milieu de référence. Dans le cas contraire, les opérations de corrections conduiront à des valeurs de capacitance relatives à celle du milieu de référence.

On va maintenant décrire le principe de traitement des signaux de capacitance brute mis en oeuvre dans le procédé selon l'invention. Pour la suite de la mise en oeuvre du procédé selon l'invention, on utilisera le principe suivant pour traiter les signaux de capacitance brute : On soustrait au signal de capacitance brute mesuré Cm_(G, f), la valeur calculée à partir du modèle Ccal(G, f) (8). On obtient une mesure de capacitance corrigée Cm_cor(G, f)

$$Cm\_cor(G, f) = Cm(G, f) - Ccal(G, f) \qquad (9)$$

Nous rappellerons que cette opération permet de corriger 90 % des erreurs dues à la fois à la polarisation systématique et aux erreurs liées aux imperfections matérielles.

Après combinaison des équations (1), (2), (4) et (6) on obtient :

$$Cm\_cor(G, f) = Cx(G, f) + Cpol\_alea(G, f) \qquad (10)$$

La mesure de capacitance corrigée est donc égale à la somme de la capacitance du milieu diélectrique étudié et de l'erreur de polarisation aléatoire.

Deuxième niveau de correction : correction de l'erreur de polarisation aléatoire

Etape 3 : détermination de la polarisation aléatoire à la fréquence $f_1$

La capacitance de polarisation aléatoire est déterminée à une seule fréquence de mesure. Elle est obtenue en soustrayant à la mesure de capacitance effectuée à basse fréquence $f_1$ une valeur calculée à partir du modèle de calibration Ccal(G, f) (8) suivant le principe de traitement des mesures de capacitance brute (cf. figure 7).

Dans le cas d'un milieu contenant des cellules biologiques, cette soustraction ne conduit qu'à une estimation de la polarisation aléatoire, car la quantité de biomasse contenue dans le milieu Cx(G, f1) influe également sur ce résultat.

On obtient :

$$Cpol\_alea(G, f_1) = Cm\_cor(G, f_1) - Cx(G, f_1) \qquad (11)$$

Si on considère que la polarisation aléatoire résulte de l'adsorption de composés qui modifient dans un sens ou dans l'autre l'épaisseur de la double couche ionique de Nernst, responsable de la polarisation systématique, on démontre aisément que la variation de la polarisation aléatoire en fonction de la fréquence de mesure et de la conductance du milieu est proportionnelle à $G^2/f^2$. (5). Cette hypothèse de comportement de polarisation aléatoire a été confirmée à partir d'expérimentations réalisées avec un appareil de mesure de biomasse du type décrit dans le document FR2812725.

On obtient alors expérimentalement la relation :

$$Cpol\_alea(G, f) = a_{alea}. \ f^p. \ G^2 \qquad (12)$$

Avec:

- G : conductance du milieu
- $a_{alea}$ : constante prédéfinie (en général égale à 1/capacitance de polarisation aléatoire)
- p : constante correspond à la pente de la polarisation (en pratique très proche de la valeur théorique égale à -2)

La fréquence de mesure de la polarisation aléatoire doit être choisie très basse pour que, grâce au terme en $1/f^2$ (12), la valeur mesurée soit la plus élevée possible devant la capacitance de la biomasse du milieu. En pratique, une fréquence de mesure de 100 Khz permet une excellente détermination de la capacitance de polarisation aléatoire.

On peut alors écrire que :

$$\mathrm{Cpol\_alea(G, f_1)} \approx \mathrm{Cm\_cor(G, f_1)} \qquad (13)$$

Etape 4 : estimation de la capacitance du milieu à la fréquence $f_2$

Une mesure de capacitance, caractéristique du milieu diélectrique étudié, est effectuée à une deuxième fréquence $f_2$. Elle est obtenue en soustrayant à la mesure de capacitance une valeur calculée à partir du modèle de calibration Ccal(G, f) (8) suivant le principe de traitement des mesures de capacitance brute. Dans le cas de mesures sur des cellules biologique, cette fréquence est choisie de préférence près de la fréquence caractéristique fc de la cellule, selon le modèle proposé par Pauly et Schwan.

Cette soustraction donne la valeur de la capacitance caractéristique du milieu diélectrique étudié, à laquelle s'ajoute encore la valeur de capacitance de polarisation aléatoire à fréquence $f_2$ :

$$\mathrm{Cm\_cor(G, f_2)} = \mathrm{Cx(G, f_2)} + \mathrm{Cpol\_alea(G, f_2)} \qquad (14)$$

Etape 5 : correction de la polarisation aléatoire sur la mesure de capacitance à fréquence $f_2$

Les équations de la capacitance de polarisation aléatoire (11) et de l'estimation de la capacitance caractéristique du milieu diélectrique étudié (14) sont ensuite combinées.

Les valeurs de la capacitance de polarisation aléatoire (12) aux deux fréquences $f_1$ et $f_2$ sont :

$$\mathrm{Cpol\_alea(G, f_1)} = a_{alea} \cdot f_1^{p} \cdot G^2$$

$$\mathrm{Cpol\_alea(G, f_2)} = a_{alea} \cdot f_2^{p} \cdot G^2$$

En divisant ces deux équations on trouve :

$$\mathrm{Cpol\_alea(G, f_2)} = \mathrm{Cpol\_alea(G, f_1)} \cdot (f_2 / f_1)^{p} \qquad (15)$$

La combinaison des équations (11), (14) et (15) conduit à l'expression suivante :

$$\mathrm{Cx(G, f_2)} = \mathrm{Cm\_cor(G, f_2)} - (f_2 / f_1)^{p} \cdot [\mathrm{Cm\_cor(G, f_1)}] + [\mathrm{Cx(G, f_1)}] \cdot (f_2 / f_1)^{p} \qquad (16)$$

La combinaison des équations (9) et (15) conduit à une équation (17) exprimée ci-après qui fournit la formule de calcul de la capacitance du milieu diélectrique étudié, corrigée de tous les effets de polarisation et d'électronique. Ce résultat est appelé dans la suite « méthode de correction FG ».

Formule générale de la méthode de correction FG :

$$\mathrm{Cx(G, f_2)} = \mathrm{Cm\_(G, f_2)} - \mathrm{Ccal(G, f_2)} - (f_2 / f_1)^{p} \cdot [\mathrm{Cm\_(G, f_1)} - \mathrm{Ccal(G, f_1)}] + [\mathrm{Cx(G, f_1)}] \cdot (f_2 / f_1)^{p} \qquad (17)$$

avec :

- Cx(G, $f_2$) : capacitance du milieu diélectrique à la fréquence $f_2$

- $Cm\_(G, f_2)$ : capacitance brute mesurée à fréquence $f_2$
- $Ccal(G, f_2)$ : capacitance fonction de G, mesurée dans un milieu de référence à la fréquence $f_2$
- $Cm\_(G, f_1)$ : capacitance brute mesuré à la fréquence $f_1$
- $Ccal(G, f_1)$ : capacitance fonction de G mesurée dans un milieu de référence à la fréquence $f_1$
- $Cx(G, f_1)$ : capacitance du milieu diélectrique à la fréquence $f_1$
- $f_1$ : fréquence où est estimée la capacitance de polarisation aléatoire
- $f_2$ : fréquence de mesure de la capacitance caractéristique du milieu diélectrique
- p : constante correspondant à la pente de la polarisation (en général égale à -2)

Dans cette formule (17), le terme $[Cx(G, f_1)]$, introduit une erreur car il n'est pas connu. Ce terme peut être rendu négligeable par un choix de fréquence de mesure de polarisation $f_1$ le plus bas possible, comme expliqué précédemment. Une fréquence de 100 KHz donne de très bons résultats.

Par exemple une fréquence de mesure $f_2$ = 1000 KHz divise par 100 ce terme d'erreur grâce au terme en facteur $(f_2/ f_1)^p$.

Les avantages de cette méthode de correction FG sont donc :

- de corriger globalement les erreurs de l'électronique et les effets de polarisation systématique et aléatoire,
- de corriger également les effets de second ordre, tels que le produit de l'effet combiné de la polarisation avec les erreurs globales de l'électronique, l'incertitude sur la valeur de la pente (fixée à -2) de la polarisation systématique, et le produit de l'effet combiné de l'aération du milieu avec les erreurs globales de l'électronique,
- d'utiliser une méthode de calibration qui permette de déterminer en une seule fois les paramètres d'un modèle de correction global, intégrant les effets de l'électronique et de la polarisation systématique, cette méthode de calibration étant effectuée, de manière automatisable, dans un milieu de référence sans cellules biologiques,
- de corriger la polarisation absolue à la fréquence $f_2$ de façon plus précise que les méthodes numériques existantes ; la correction est moins influencée par une erreur sur la pente de la polarisation p ou une dérive des fréquences f1 ou f2, car le modèle électrochimique de type $a.f^p.G^2$ n'est appliqué qu'à la polarisation aléatoire, laquelle ne présente qu'une proportion relativement faible (environ 10 % au maximum) de la polarisation absolue ;
- de mettre en oeuvre une formule de correction (17) qui n'utilise que des opérateurs simples et donc facilement implantables dans un système embarqué, et
- de n'utiliser qu'une seule fréquence pour déterminer la capacitance de polarisation aléatoire.

Des améliorations de la qualité de cette méthode de correction peuvent être envisagées. Nous allons montrer que la formule de la méthode de correction FG (17) peut être modifiée suivant le type de diélectrique que l'on souhaite mesurer, en présentant deux exemples non limitatifs de mise en oeuvre de la méthode de correction FG.

Exemple 1 : Si la capacitance du milieu diélectrique mesuré ne varie pas en fonction de la fréquence, alors :

$$Cx(G, f_2) = Cx(G, f_1)$$

La formule (17) se simplifie alors, en Formule de la méthode de correction FG_B :

$$C_{x\_FG\_B}(G, f_2) = [Cm\_(G, f_2) - Ccal(G, f_2) - (f_2/ f_1)^p \cdot [Cm\_(G, f_1) - Ccal(G, f_1)]\, ]/ [1-(f_2/ f_1)^p] \qquad (18)$$

Tous les termes de cette formule FG_B sont connus explicitement.

Exemple 2: Si le milieu diélectrique à mesurer est une suspension de cellules biologiques, la formule de la méthode de correction FG (17) peut se mettre sous la forme :

$$Cx(G, f_2).[1- (f_2/ f_1)^p.Cx(G, f_1)/Cx(G, f_2)] = Cm\_(G, f_2) - Ccal(G, f_2) - (f_2/ f_1)^p \cdot [Cm\_(G, f_1) - Ccal(G, f_1)] \qquad (19)$$

En utilisant le modèle de Pauly et Schwann et en considérant que la capacitance du milieu est négligeable devant la capacitance mesurée, on a :

$$Cx(G, f_1) = \Delta C_{cell} / ( 1 + (f_1/f_c)^2 )$$

$$Cx(G, f_2) = \Delta C_{cell} / ( 1 + (f_2/f_c)^2 )$$

avec fc : fréquence caractéristique de la cellule biologique

En divisant ces deux équations, on trouve que :

$$Cx(G, f_1) / Cx(G, f_2) = ( 1 + (f_2/f_c)^2 )/ ( 1 + (f_1/f_c)^2 ) \qquad (20)$$

La combinaison des équations (19) et (20) et d'une pente égale à -2 conduit à une formule de méthode de correction FG_C :

$$C_{x\_FG\_C} (G, f_2) = (1 + f_1^2/f_c^2) \cdot 1/(1 - f_1^2/f_2^2) \cdot [\, Cm\_(G, f_2) - Ccal(G, f_2) - (f_2/ f_1)^p \cdot [Cm\_(G, f_1) - Ccal(G, f_1) \,]\,] \quad (21)$$

Cette formule, adaptée aux milieux biologiques, est significativement plus efficace que la méthode FG de base (17). Le terme de correction principal pour le milieu biologique est en pratique $1/(1 - f_1^2/f_2^2)$. Il correspond à l'hypothèse d'égalité de valeur pour les deux capacitances mesurées à fréquence $f_1$ et $f_2$, comme dans l'exemple précédent.

Le terme $(1 + f_1^2/f_c^2)$ apporte une correction moins significative. Il peut être remplacé par 1 dans la majorité des cas. Une autre solution est de lui donner une valeur indicative. Pour les cas où il serait utilisé pour affiner la mesure, il est nécessaire de connaître la fréquence fc caractéristique du milieu utilisé,

- soit en la calculant à partir de l'équation théorique

$$fc = 1/(4.\pi.r.Cm.(1/\sigma c - 1/\sigma m))$$

   dans laquelle $\sigma m$ est la conductivité du milieu, déterminée à partir de la conductance G mesurée par l'appareil, les autres paramètres Cm et $\sigma c$ sont estimés à partir de la littérature scientifique (respectivement 1 $\mu$F/cm et 3 mS/cm), et r est le rayon des cellules biologiques étudiées, supposées sphériques.
- soit par une méthode en ligne permettant l'évaluation de fc à partir de mesures à différentes fréquences, corrigées par la méthode FG. Les mesures de capacitance et l'évaluation de fc peuvent alors être affinées en quelques itérations.

Troisième niveau de correction : correction des dérives d'offset et des variations de capacitances du milieu (hors biomasse)

Etape 6 : Suppression de la dérive d'offset de l'électronique

Une fois que le signal de capacitance est corrigé par la méthode FG, il subsiste une légère dérive thermique de ce signal qui est due à la dérive d'offset de l'électronique. Cette erreur est corrigée à l'aide de la mesure de capacitance faite à une troisième fréquence. On utilise pour cela la fréquence la plus élevée de l'appareil (par exemple, 10 MHz) car, dans ce cas, elle offre l'avantage de supprimer également toute variation de capacitance du milieu diélectrique qui serait liée à une évolution du milieu de suspension des cellules biologiques.

Suivant le principe de traitement des mesures de capacitance brute, on obtient le terme corrigé :

$$Cm\_cor(G, f_3) = Cm(G, f_3) - Ccal(G, f_3)$$

Ce terme de correction doit être soustrait de la méthode de correction FG que l'on souhaite utiliser.

Par exemple, à partir de la méthode de correction FG_C (21), on aboutit à la méthode de correction FG_C0 :

$$\Delta C_x(G, f_2) = (1 + f_1^2/f_c^2) \cdot 1/(1 - f_1^2/f_2^2) \cdot [ \, Cm\_(G, f_2) - Ccal(G, f_2) - (f_2/f_1)^p \cdot [Cm\_(G, f_1) - Ccal(G, f_1)] \, ] - $$
$$(Cm\_(G, f_3) - Ccal(G, f_3)) \qquad (22)$$

avec :

- $\Delta Cx(G, f_2)$ : incrément capacitif du milieu diélectrique à la fréquence $f_2$
- $Cm\_(G, f_2)$ : capacitance brute mesurée à fréquence $f_2$
- $Ccal(G, f_2)$ : capacitance fonction de G, mesurée dans un milieu de référence à la fréqueuce $f_2$
- $Cm\_(G, f_1)$ : capacitance brute mesurée à la fréquence $f_1$
- $Ccal(G, f_1)$ : capacitance fonction de G mesurée dans un milieu de référence à la fréquence $f_1$
- $Cx(G, f_1)$ : capacitance du milieu diélectrique à la fréquence $f_1$
- $f_1$ : fréquence où est estimée la capacitance de polarisation aléatoire
- $f_2$ : fréquence de mesure de la capacitance caractéristique du milieu diélectrique
- $p$ : constante correspondant à la pente de la polarisation (en général égale à -2)
- $f_3$ : fréquence où est mesurée la dérive thermique de l'offset électronique et la capacitance du milieu diélectrique (hors cellules)
- $f_c$ : fréquence caractéristique (Pauly et Schwann) du milieu biologique étudié

Cette formule de méthode de correction FG_C0 a été implantée dans un appareil de mesure de biomasse du type de celui décrit dans le document FR2812725. Cette formule permet de corriger :

- l'ensemble des erreurs de capacitance, fonctions de la fréquence et de la conductance, de l'électronique, des effets de ligne, et du capteur,
- les erreurs de polarisations systématiques et aléatoires,
- la dérive thermique de l'offset de l'électronique,
- la variation de la capacitance du milieu diélectrique (hors cellules biologiques).

Etape 7 : détermination de la permittivité et de la conductance du milieu diélectrique

Les valeurs de capacitances et de conductances mesurées sont converties respectivement en permittivité absolue et en conductivité en multipliant les valeurs de capacitance et les valeurs de conductance par un facteur de sonde k.

$$\varepsilon = C \cdot k \qquad et \qquad \sigma = G \cdot k \qquad (23)$$

Ce facteur k est constant pour une géométrie donnée de capteur. Il est égal à L/S dans le cas d'un condensateur plan ayant une surface S d'électrode et une distance L inter électrodes.

Dans le cas de l'appareil de mesure de biomasse du type décrit dans le document FR2812725, le facteur k est déterminé expérimentalement à l'aide d'une solution d'eau salée de conductivité connue. La détermination du facteur k est obtenue en divisant la valeur de conductivité de la solution par la conductance mesurée par l'appareil.

Ce facteur de sonde k peut être appliqué aux mesures de conductance et de capacitance avant ou après la correction de ces mesures par la méthode de correction FG.

Appliqué à la valeur de $\Delta C_x(G, f_2)$ obtenue précédemment (Equation 22), il permet d'obtenir l'incrément de permittivité $\Delta\varepsilon$ avec

$$\Delta\varepsilon = \Delta C_x(G, f_2) \cdot k \qquad (24)$$

Etape 8 : détermination de la concentration de biomasse

L'amplitude de la dispersion $\beta$, pour des cellules supposées sphériques, tant que la fraction volumique P n'est pas trop forte (comme c'est le cas dans la plupart des fermentations) est donnée par la relation classique :

$$\Delta\varepsilon \approx \frac{9}{4} r\, C_m\, P \qquad (25)$$

dans laquelle $\Delta\varepsilon$ représente ici l'incrément de permittivité calculé en faisant la différence entre permittivité à fréquence basse et permittivité à fréquence élevée, de part et d'autre de la dispersion $\beta$. Cette relation est également valable lorsque la permittivité "basse" est prise à une fréquence quelconque au dessous de la fréquence haute, dans le domaine de la dispersion $\beta$, et en particulier à la fréquence f2, telle que définie précédemment.

Pour une biomasse microbienne, on peut considérer qu'il y a proportionnalité entre la concentration de biomasse X et la fraction volumique P, en faisant l'hypothèse que la capacitance membranaire Cm et le rayon r sont des constantes. On obtient ainsi la relation

$$X = \gamma \,.\, \Delta\varepsilon \qquad (26)$$

avec :

$\gamma$ : coefficient de cellule biologique ; ce coefficient peut être déduit à partir d'un étalonnage dans une suspension de biomasse dont la concentration est connue ;

$\Delta\varepsilon$ : variation de permittivité calculée selon l'équation (24)

Etape 9 : correction des effets d'aération sur les mesures de permittivité et de conductance

Dans un milieu aéré, le facteur de sonde k (k=L/S dans le cas d'un condensateur plan S surface d'électrode, L distance inter électrodes) augmente car la surface apparente des électrodes est réduite par la présence des bulles. La longueur du parcours des lignes de champs inter-électrodes est quant à elle augmentée, mais de façon moins significative.

Pour un milieu diélectrique de permittivité $\varepsilon$ et de conductivité $\sigma$, la capacitance et la conductance mesurées sont exprimées, respectivement sans aération et avec aération, de la façon suivante :

sans aération :

$$C_0(G_0,\, f) = \varepsilon \,/\, k \quad \text{et} \quad G_0 = \sigma \,/\, k \qquad (27)$$

avec aération :

$$C_a(G_a,\, f) = \varepsilon \,/\, k_a \quad \text{et} \quad G_a = \sigma \,/\, k_a \qquad (28)$$

La combinaison des équations (27) et (28) donne :

$$k_a = k \,.\, C_0(G_0,\, f) \,/\, C_a(G_a,\, f) \quad = k \,.\, G_0 \,/\, G_a \qquad (29)$$

En choisissant une fréquence prédéterminée $f_4$ où la valeur des caractéristiques diélectriques du milieu est la plus stable quels que soient les changements de paramètres d'environnement, il possible de déterminer une variation de capacitance liée uniquement au changement du taux d'aération. Pour un milieu contenant des cellules biologiques en suspension, cette fréquence est à choisir nettement au-dessus de la fréquence fc, typiquement entre 10 et 100 MHz.

Cette variation de capacitance est alors obtenue en soustrayant à la mesure de capacitance effectuée à la fréquence $f_4$, la valeur calculée à partir du modèle de calibration Ccal(G,$f_4$) (8), suivant le principe de traitement des mesures de capacitance brute Cm_cor(G,$f_4$).

$$Cm\_cor(G_a,\, f_4) = C_a(G_a,\, f_4) - C_0(G_0,\, f_4) \qquad (30)$$

La combinaison des équations (26) et (27) donne le facteur de sonde en milieu aéré :

$$k_a = k / [1 + (Cm\_cor(G_a, f_4) / C_0(G_0, f_4))] \qquad (31)$$

avec :

- k : facteur de sonde d'un milieu de référence non-aéré
- $C_0(G, f_4)$ capacitance du milieu de référence non-aéré et à la fréquence $f_4$. Cette valeur prédéterminée est obtenue, soit par un abaque de permittivité des matériaux diélectriques et en utilisant le facteur de sonde k, soit en mesurant la capacitance du milieu de référence non aéré suivant le principe de traitement des mesures de capacitance brute $Cm\_cor(G_0, f_4)$. En général, la permittivité ou la capacitance du milieu de référence est celle de l'eau.

Le facteur de sonde en milieu aéré $k_a$ est ensuite utilisé pour déterminer la permittivité du milieu diélectrique, corrigée des effets d'aération sur la mesure de capacitance (32). La conductivité du milieu est également déterminée en corrigeant les effets de l'aération sur la mesure de conductance (33).

La combinaison des équations (25) et (28) donne la permittivité du milieu à toutes les fréquences de l'appareil :

$$\varepsilon = Cm\_cor(G_a, f) \ . \ k \ / [1 + (Cm\_cor(G_a, f_4) / C_0(G_0, f_4))] \qquad (32)$$

La conductivité du milieu à toutes les fréquences de l'appareil est exprimée de la façon suivante :

$$\sigma = G_a \ . \ k / [1 + (Cm\_cor(G_a, f_4) / C_0(G_0, f_4))] \qquad (33)$$

avec :

$\varepsilon$ : permittivité déterminée à la fréquence f et corrigée des effets d'aération
$\sigma$ : conductivité déterminée à la fréquence f et corrigée des effets d'aération
$Cm\_cor(G, f)$ : capacitance mesurée suivant le principe de traitement des mesures de capacitance, affectée de l'aération et mesurée la fréquence f
$Cm\_cor(G, f_4)$ : capacitance mesurée suivant le principe de traitement des mesures de capacitance, affectée de l'aération et mesurée à la fréquence de détermination d'aération $f_4$
$C_0(G_0, f_4)$ : Capacitance du milieu de référence dans des conditions de non-aération et à la fréquence $f_4$.
$G_a$ : conductance du milieu affecté de l'aération et mesurée à fréquence $f_4$
$G_0$ : conductance du milieu du milieu de référence dans des conditions de non-aération

Les valeurs de capacitances et conductances corrigées de l'aération peuvent être déterminées avec l'équation (27).

Etape 10 : Détermination des paramètres caractéristiques d'une dispersion diélectrique

La méthode la plus souvent utilisée pour l'évaluation de la biomasse, qui consiste à mesurer la capacitance du milieu à une ou deux fréquences prédéfinies, ne permet pas en réalité une évaluation directe du biovolume. C'est en fait le produit P.r.Cm, grandeur complexe, qui est évaluée par l'incrément $\Delta\varepsilon$, qui dépend simultanément de la conductivité du milieu, de la taille et de la distribution de taille des cellules, ainsi que de leur état physiologique. Pour évaluer avec plus de vraisemblance le biovolume, il est nécessaire d'extraire de la courbe de dispersion suffisamment d'information pour identifier le plus grand nombre possible de variables. La courbe de dispersion $\beta$ peut être obtenue en appliquant la méthode de correction des mesures de capacitances précédemment décrite.

On expose ici, dans le cadre de la présente invention, une méthode permettant de retrouver les descripteurs mathématiques de la dispersion $\beta$, en utilisant pour cela une technique d'ajustement linéaire, alors que les autre méthodes proposées utilisent des techniques d'ajustement non-linéaires, potentiellement plus précises, mais beaucoup plus lourdes à mettre en oeuvre, en particulier si elles doivent être implantées dans des systèmes à base de microcontrôleurs.

On utilise un polynôme d'ordre n pour représenter la courbe Cx = f(fréquence). On peut écrire :

$$Cx = P(f) = a0 + a1. \ f + a2. \ f2 + a3. \ f3 + ..... \ an. \ fn$$

Un fait remarquable est qu'à partir d'un ordre n égal ou supérieur à 2, la fonction polynomiale peut être ajustée sur la courbe de dispersion β, de part et d'autre de la fréquence fc, comme l'illustre la figure 8. En effet, l'inflexion de la courbe de dispersion n'existe qu'en coordonnées semi-logarithmiques. Du coté des fréquences élevées, le polynôme présente toujours une branche parabolique, qui tend vers l'infini (+). En principe, ce type de fonction ne peut donc pas être appliqué directement à l'étude du domaine des fréquences les plus élevées, largement supérieures à fc. En revanche, il est impératif que les mesures encadrent bien cette fréquence caractéristique, puisque c'est elle qui conditionne la détermination du point d'inflexion de la courbe de dispersion β. Le polynôme décrit très bien le domaine des fréquences basses et peut en particulier être calculé en f = 0, où

$$Cx_{(f=0)} = a0$$

On calcule ainsi aisément la valeur de l'incrément capacitif.

On va maintenant décrire de manière détaillée la procédure à appliquer pour le calcul des différents coefficients du polynôme. On se bornera ici à la présentation du cas où n = 2. Il s'agit d'identifier les trois coefficients de la fonction :

$$Cx = a0 + a1\ f + a2\ f^2$$

On a donc besoin d'au moins trois mesures réalisées à trois fréquences réparties autour de la fréquence caractéristique typique de la dispersion β. Les données publiées dans la littérature scientifique montrent que cette fréquence caractéristique est souvent comprise entre 0.5 et 2 MHz. Puisque la mesure doit être extrapolée à f=0, il est souhaitable de fixer une fréquence de mesure à la plus faible valeur techniquement envisageable (typiquement 0.1 à 0.3 MHz), afin de limiter l'erreur liée à l'extrapolation. D'autre part, on a observé que la fonction polynomiale représentait mal le domaine des fréquences élevées. On utilisera donc le plus souvent une fréquence haute égale à 2 ou 3 MHz. Cependant, dans le cas de cellules de petite taille (bactéries par exemple), pour lesquelles la fréquence caractéristique est plus élevée, il faudra choisir une fréquence de mesure également plus élevée. Le domaine de fréquence pourrait être étendu en utilisant une fonction polynomiale d'ordre supérieur, au prix d'une complexité plus importante et d'une réduction de la "robustesse" de la méthode.

La méthode se ramène ainsi à la résolution d'un système de trois équations (les trois mesures) à trois inconnues (les trois paramètres). Sous forme matricielle, le problème s'écrit :

$$[C] = [F]\ x\ [Coeffs],$$

qui se résout en [Coeffs] = [F]$^{-1}$.C

Les coefficients de la matrice inverse F$^{-1}$, qui ne dépendent que des valeurs des fréquences utilisées, sont calculés au préalable. La méthode se ramène donc au calcul d'une série de multiplications.

Ainsi, par exemple pour des fréquences de mesure de 0.300, 1 et 3 MHz, les valeurs numériques des différents éléments des tableaux sont les suivantes :

$$\begin{bmatrix} a0 \\ a1 \\ a2 \end{bmatrix} = \begin{bmatrix} 1.5873 & -0.6429 & 0.0556 \\ -2.1164 & 2.3571 & -0.2407 \\ 0.5291 & -0.7143 & 0.1852 \end{bmatrix} x \begin{bmatrix} Cx(f5) \\ Cx(f6) \\ Cx(f7) \end{bmatrix}$$

Afin d'améliorer la robustesse de la méthode, on peut être amené à réaliser un nombre plus grand de mesures, à des fréquences supplémentaires. On utilise alors une méthode similaire, dans laquelle la matrice inverse est remplacée par une matrice dite "pseudo-inverse", dont les coefficients sont également pré-calculés.

La dispersion est observée en faisant le changement de variable z = Ln f, soit f= e$^z$.

$$Cx = a0 + a1\ e^z + a2\ e^{2z} + a3\ e^{3z} + \ldots + an\ e^{nz}$$

On trouve la fréquence caractéristique fc en cherchant la position du point d'inflexion. On a :

$$dCx/dz = a_0 + a_1\,e^z + a_2\,e^{2z} + a_3\,e^{3z} + \ldots + a_n\,e^{nz}$$

$$d^2Cx/dz^2 = a1\,e^z + 4\,a2\,e^{2z} + 9\,a3\,e^{3z} + \ldots + n^2\,an\,e^{nz}$$

Le point d'inflexion est trouvé lorsque la dérivée seconde est nulle, ce qui conduit à :

$$d^2Cx/dz^2 = 0 = a1 + 4\,a2\,e^z + 9\,a3\,e^{2z} + \ldots + n^2\,an\,e^{(n-1)z}$$

En effectuant le changement de variables inverse, on obtient :

$$0 = a1 + 4\,a2\,fc + 9\,a3\,fc^2 + \ldots + n^2\,an\,fc^{(n-1)}$$

Pour un polynôme P(f) de degré 2, la relation se ramène à :

$$0 = a1 + 4\,a2\,fc,$$

soit fc = -a1/(4 a2)

Il faut impérativement que la concavité du polynôme de degré 2 soit orientée vers le bas, c'est à dire que le coefficient a2 soit positif. Dans le cas contraire, la fréquence correspondant au point d'inflexion est négative, c'est à dire que la fréquence caractéristique est en fait trop élevée par rapport au domaine de fréquences de mesure pour qu'une parabole puisse être ajustée aux mesures.

Alternativement, on peut utiliser un polynôme P(f) de degré 3. Le calcul est à peine plus engagé : on obtient par dérivation

$$0 = a1 + 4\,a2\,fc + 9\,a3\,fc^2,$$

équation du second degré qui conduit à

$$\Delta' = 4\,a2^2 - 9\,a1\,a3,$$

d'où on tire

$$fc = [-2a2 \pm (4\,a2^2 - 9\,a1\,a3)^{1/2}]/(9\,a3)$$

En revanche, pour des polynômes de degré supérieur, il serait nécessaire de réaliser une résolution numérique, par exemple en utilisant la méthode de Newton-Raphson. Se poserait alors le problème du repérage de la solution pertinente.

La recherche de la position du point d'inflexion repose sur un processus itératif tel que fi+1 = fi - Pi/P'i

On dérive donc une fois de plus le polynôme. On obtient

$$P'i = 4\,a2 + 18\,a3\,fc + \ldots + (n-1)\,n^2\,an\,fc^{(n-2)}$$

Pour un polynôme de degré 3, la formule d'itération se ramène à :

$$fi+1 = fi - Pi/P'i = fi - (a1 + 4\,a2\,fi + 9\,a3\,fi^2)\,/\,(4\,a2 + 18\,a3\,fi)$$

La solution est généralement atteinte avec une précision acceptable en trois itérations. Le problème principal est de déterminer des conditions initiales suffisamment proches de la solution vers laquelle on doit converger. On peut démarrer de la fréquence de mesure la plus basse utilisée fmin (0.3 MHz), mais dans certains cas (fc élevé), l'algorithme converge alors vers un point d'inflexion situé à fc < 0. Il faut alors redémarrer l'itération à partir de la valeur de fréquence la plus élevée utilisée fmax.

Le coefficient $\alpha$ du milieu diélectrique, est obtenu en calculant la pente de la courbe de dispersion au voisinage de la fréquence caractéristique fc. A partir de la relation de Cole-Cole, la pente au point d'inflexion est donnée par la relation :

$$(dCx/df)_{fc} = -a0 \, (1 - \beta) \, / \, (2 \, fc \, (1 + \sin(\pi.\alpha/2)))$$

Cette relation n'est pas inversible, c'est à dire qu'on ne .peut pas obtenir analytiquement une valeur de $\alpha$. Pour y parvenir, une autre approche consiste à montrer que la fonction de dispersion en fonction de la fréquence établie empiriquement par Cole et Cole peut être avantageusement remplacée par la fonction :

$$Cx = Cx_{high} + \Delta Cx \, / \, (1 + (f/fc)^{2(1-b)})$$

dans laquelle le coefficient $\beta$ est compris entre 0 et 1. Cette nouvelle relation n'est pas intrinsèquement meilleure ou moins bonne que celle de Cole et Cole, puisqu'elle est également empirique. Il n'est cependant pas possible de discerner expérimentalement une relation de l'autre. Cette nouvelle relation permet le calcul analytique du coefficient $\beta$.

On a en effet la relation suivante :

$$(dCx/df)_{fc} = - a0 \, (1-\beta) \, / \, (2 \, fc) = a1 + 2 \, a2 \, fc + 3 \, a3 \, fc^2 + ... + n \, an \, fc^{(n-1)}$$

d'où on tire ensuite $\beta$ :

$$\beta = 1 + 2 \, fc \, (a1 + 2 \, a2 \, fc + 3 \, a3 \, fc^2 + ... + n \, an \, fc^{(n-1)}) \, / \, a0$$

Une relation empirique permet, si cela était nécessaire, de recalculer la valeur du coefficient $\alpha$ à partir de celle de $\beta$. On a établi en effet aisément la relation approchée suivante :

$$\alpha = (0.627314 \, \beta^2 - 0.061700 \, \beta + 0.439407) \, \beta$$

expression qui, pour des valeurs de $\alpha$ comprises entre 0 et 0.5, domaine habituel de variation, peut être affinée en

$$\alpha = (0.495698 \, \beta^2 - 0.062162 \, \beta + 0.413456) \, \beta$$

Alternativement, en raison du caractère symétrique de la dispersion $\beta$ autour du point d'inflexion correspondant à la fréquence caractéristique fc, il est possible d'utiliser les fréquences les plus élevées, toujours en encadrant la fréquence fc. Il faut alors effectuer un changement de variables, en posant h = 1/f et hc = 1/fc. Le traitement mathématique est ensuite réalisé comme exposé précédemment, mais conduit à trouver Cfhigh, en extrapolant le polynôme utilisé à h = 0. On obtient fc en inversant la valeur de hc trouvée.

Cette variante est particulièrement intéressante parce que les erreurs associées aux polarisations systématiques et aléatoires n'affectent que très peu les mesures de capacitances réalisées aux fréquences élevées. Par ailleurs, on constate expérimentalement que le bruit de mesure est également plus réduit, ce qui permet d'obtenir des valeurs des descripteurs également moins bruitées.

On a ainsi obtenu la valeur des trois descripteurs caractérisant la dispersion $\beta$. On peut maintenant utiliser cette information pour améliorer la mesure de biomasse. En effet, nous avons vu que la mesure de capacitance renvoyait une valeur proportionnelle au produit P.r.Cm, qui est donc dépendante de la taille des cellules. Nous avons vu également que la valeur de fc est inversement proportionnelles au produit r.Cm. En effectuant le produit C.fc, on élimine donc les variables inconnues r et Cm, et on obtient une valeur indépendante de la capacitance membranaire et surtout de la taille, qui peut varier dans des proportions fortes. On obtient :

$$P = \frac{2}{9}\,\Delta\varepsilon \cdot f_c\,\pi\;\frac{1}{\sigma_c} + \frac{1}{2\,\sigma_m}$$

Dans cette expression, la fraction volumique de biomasse P ne dépend plus que d'une seule variable inconnue σc, puisque σm peut être mesurée par l'appareil de mesure de biomasse en même temps que la capacitance. Comme σc est une variable qui est métaboliquement régulée, et qu'il a été montré expérimentalement qu'elle était remarquablement insensible aux variations de la conductivité σm du milieu, on peut ainsi parvenir à une évaluation de la fraction volumique P nettement améliorée par rapport à la simple mesure de l'incrément diélectrique.

Bien sûr, l'invention n'est pas limitée aux exemples qui viennent d'être décrits et de nombreux aménagements peuvent être apportés à ces exemples sans sortir du cadre de l'invention. En particulier, le procédé de détermination selon l'invention peut être mis en oeuvre dans d'autres appareils de mesure de biomasse que celui décrit dans le document FR2812725.

**Revendications**

1. Procédé d'évaluation d'une biomasse dans un milieu diélectrique à mesurer, notamment de cellules en suspension dans un fluide, dans lequel les mesures de capacitance et de conductance sont effectuées au moyen d'un appareil comprenant des électrodes en contact avec le milieu et des moyens électroniques de traitement des signaux de capacitance et de conductance prélevés à partir desdites électrodes, ce signal de capacitance étant entaché d'erreurs de capacitance dues à une polarisation aléatoire, d'erreurs de capacitance dues à une polarisation systématique, et d'erreurs de capacitances dues aux erreurs globales provenant des moyens électroniques,

   - les erreurs de capacitance dues à la polarisation aléatoire étant corrigées séparément de celles dues à la polarisation systématique et de celles dues à électronique,
   - le procédé comprend en outre (i) une modélisation globale de la polarisation systématique et des erreurs de capacitance dues aux erreurs globales de l'électronique sous la forme d'une équation commune du modèle d'erreur de capacitance ($C_{cal}(G, f)$) fonction de la conductance (G) du milieu et de la fréquence d'excitation (f) des électrodes conductrices, et (ii) une détermination de valeurs de capacitance corrigées (Cm_cor(G, f)) en comparant chaque mesure de capacitance brute (Cm(G, f)) provenant de l'appareil et effectuée à une fréquence prédéterminée (f) à la valeur de ladite équation commune du modèle d'erreur de capacitance ($C_{cal}(G, f)$) à ladite fréquence prédéterminée (f),
   - l'évaluation de la biomasse (X) étant obtenue à partir d'une différence ($\Delta C_X(G,f_2)$) entre un premier signal de capacitance du milieu ($C_{X\_FG\_C}(G,f_2)$) obtenu à partir d'une première mesure de capacitance brute (Cm_(G, $f_2$)) dudit milieu mesurée à une première fréquence ($f_2$), et un second signal de capacitance corrigée (Cm_cor(G,$f_3$)) obtenu à partir d'une seconde mesure de capacitance brute (Cm_(G, $f_3$)) dudit milieu mesurée à une seconde fréquence ($f_3$),

   le procédé comprenant une correction séparée de chacun des signaux de capacitance brute (Cm_(G, $f_2$), (Cm_(G, $f_3$)) selon au moins un premier niveau de correction, ce premier niveau de correction comprenant une correction desdits signaux de capacitance brute selon ledit modèle d'erreur de capacitance ($C_{cal}(G, f)$) dépendant de la conductance (G) du milieu auxdites première et seconde fréquences, de telle sorte à obtenir respectivement des premiers et des seconds signaux de capacitance corrigés (Cm_cor(G,$f_2$), Cm_cor(G,$f_3$)).

2. Procédé selon la revendication 1, **caractérisé en ce que** l'évaluation de la biomasse comprend une évaluation d'une concentration de la biomasse dans le milieu.

3. Procédé selon la revendication 1 ou 2, **caractérisé en ce que** l'évaluation de la biomasse comprend une évaluation d'une taille de microorganismes dans le milieu.

4. Procédé selon l'une quelconque des revendications précédentes, **caractérisé en ce que** l'évaluation de la biomasse comprend une évaluation d'une fraction volumique de la biomasse dans le milieu.

5. Procédé selon l'une quelconque des revendications précédentes, **caractérisé en ce qu'**il comprend en outre un second niveau de correction comprenant une correction desdits premier et second signaux de capacitance corrigés, à partir d'une troisième mesure de capacitance ($Cm\_(G, f_1)$) réalisée à une troisième fréquence ($f_1$), elle-même corrigée par une mesure de conductance réalisée à ladite troisième fréquence ($Ccal(G, f_1)$).

6. Procédé selon la revendication 5, mis en oeuvre pour un milieu comprenant des cellules en suspension dans un milieu, **caractérisé en ce qu'**il comprend en outre un troisième niveau de correction utilisant un modèle de comportement de la β-dispersion dans ledit milieu.

7. Procédé selon l'une quelconque des revendications précédentes, **caractérisé en ce que** l'équation commune du modèle $C_{cal}(G, f)$ est de forme polynomiale ou peut être approximée par une forme polynomiale.

8. Procédé selon l'une quelconque des revendications précédentes, **caractérisé en ce que** l'équation commune du modèle $C_{cal}(G, f)$ de forme polynomiale est d'ordre 3 ou 4.

9. Procédé selon l'une quelconque des revendications précédentes, **caractérisé en ce que** l'équation commune du modèle $C_{cal}(G, f)$ présente des coefficients calculés pour une pluralité de fréquences prédéterminées utilisées par l'appareil.

10. Procédé selon l'une quelconque des revendications précédentes, **caractérisé en ce que** les erreurs de capacitance corrigées par l'équation commune du modèle $C_{cal}(G, f)$ comprennent des erreurs capacitives fonction de la conductance et de la fréquence.

11. Procédé selon l'une quelconque des revendications précédentes, **caractérisé en ce que** les erreurs de capacitance corrigées par l'équation commune du modèle $C_{cal}(G, f)$ comprennent des erreurs liées à des effets de ligne.

12. Procédé selon l'une quelconque des revendications précédentes, **caractérisé en ce que** les erreurs de capacitance corrigées par l'équation commune du modèle $C_{cal}(G, f)$ comprennent des erreurs liées à des imperfections du capteur.

13. Procédé selon l'une quelconque des revendications précédentes, **caractérisé en ce que** le modèle de correction d'équation commune $C_{cal}(G, f)$ élimine l'incertitude sur la pente d'évolution de la polarisation systématique en fonction de la fréquence d'excitation.

14. Procédé selon l'une quelconque des revendications précédentes, **caractérisé en ce que** le modèle de correction d'équation commune $C_{cal}(G, f)$ élimine le produit de l'effet combiné de l'aération du milieu et des erreurs globales de l'électronique au sein de l'appareil.

15. Procédé selon l'une quelconque des revendications précédentes et la revendication 9, **caractérisé en ce que** les coefficients du modèle de correction $C_{cal}(G, f)$ sont déterminés à partir d'une opération de calibration dans un milieu de référence ne contenant pas de cellules biologiques et dont la conductance est modifiée de façon à parcourir la pleine échelle de la gamme de conductance de l'appareil.

16. Procédé selon l'une quelconque des revendications précédentes, **caractérisé en ce que** la détermination de l'erreur de capacitance due à la polarisation aléatoire est effectuée à une fréquence prédéterminée $f_1$ choisie la plus basse possible de sorte que la détermination de l'erreur de capacitance de polarisation aléatoire soit peu influencée par la capacitance du milieu.

17. Procédé selon la revendication 16, **caractérisé en ce que** la capacitance de polarisation aléatoire $Cm\_cor(G, f_1)$ est calculée à la fréquence $f_1$ par comparaison entre la mesure de capacitance brute issue de l'appareil et le modèle de correction $Ccal(G, f_1)$.

18. Procédé selon l'une des revendications 16 ou 17, **caractérisé en ce qu'**il comprend en outre une détermination de la capacitance du milieu diélectrique à une deuxième fréquence prédéterminée $f_2$.

19. Procédé selon la revendication 18, mis en oeuvre pour une mesure sur une suspension de cellules biologiques, **caractérisé en ce que** la deuxième fréquence prédéterminée $f_2$ est choisie proche de la fréquence caractéristique fc de ladite suspension, caractéristique de la dispersion β des cellules en suspension.

20. Procédé selon l'une des revendications 18 ou 19, **caractérisé en ce que** la capacitance du milieu diélectrique $Cm\_cor(G, f_2)$ est calculée à la fréquence $f_2$, par comparaison entre la mesure de capacitance brute issue de l'appareil et le modèle de conductance Ccal(G, $f_2$).

21. Procédé selon l'une quelconque des revendications précédentes, **caractérisé en ce qu'**il comprend en outre une modélisation de la polarisation aléatoire selon un modèle de comportement tel que $a_{alea} \cdot f^p \cdot G^2$, dans lequel :

 - G est la conductance du milieu,
 - $a_{alea}$ est une constante prédéfinie,
 - p est la pente de la polarisation.

22. Procédé selon les revendications 16, 18 et 21, **caractérisé en ce que** la détermination de la capacitance du milieu diélectrique implémente un modèle de correction résultant d'une combinaison :

 - de la mesure de capacitance de la polarisation aléatoire Cm_cor(G, $f_1$), estimée à la première fréquence prédéterminée $f_1$,
 - de la mesure de capacitance du milieu Cm_cor(G, $f_2$), estimée à la deuxième fréquence prédéterminée $f_2$,
 - et du modèle de comportement de la polarisation aléatoire $a_{alea} \cdot f^p \cdot G^2$.

23. Procédé selon la revendication 22, mis en oeuvre pour la mesure de suspensions de cellules biologiques suivant un modèle de comportement du type

$$\Delta\text{capacitance } Cx(G, f) = \Delta C_{cell} \times 1 / \left( 1 + (f/f_c)^2 \right)$$

où $f_c$ est la fréquence caractéristique du milieu.

24. Procédé selon la revendication 23, **caractérisé en ce que** la fréquence caractéristique $f_c$ est prédéterminée à partir d'un abaque.

25. Procédé selon la revendication 23, **caractérisé en ce que** la fréquence caractéristique $f_c$ est déterminée en ligne par une méthode de détermination des paramètres caractéristiques de la dispersion $\beta$.

26. Procédé selon l'une des revendications 23 à 25, **caractérisé en ce que** le diélectrique mesuré à la première fréquence prédéterminée $f_1$ est sensiblement identique au diélectrique mesuré à la seconde fréquence prédéterminée $f_2$.

27. Procédé selon l'une des revendications 23 à 26, mis en oeuvre pour la mesure d'un milieu contenant des cellules biologiques, **caractérisé en ce qu'**il comprend en outre une détermination d'une erreur de capacitance Cm_cor(G, $f_3$) due à la dérive thermique de l'offset de l'électronique et à des variations de la capacitance du milieu diélectrique de suspension, à une troisième fréquence prédéterminée $f_3$.

28. Procédé selon la revendication 27, **caractérisé en ce que** l'erreur de capacitance Cm_cor(G, $f_3$) est calculée à la troisième fréquence prédéterminée $f_3$, par comparaison entre la mesure de capacitance brute issue de l'appareil et le modèle de correction Ccal(G, $f_3$).

29. Procédé selon la revendication 28, **caractérisé en ce qu'**il comprend en outre une correction de mesures de capacitances brutes ou corrigées, entachées d'erreurs dues à la dérive thermique de l'offset de l'électronique et à des variations de capacitance du milieu de suspension, en soustrayant de ces mesures l'erreur de capacitance Cm_cor(G, $f_3$).

30. Procédé selon l'une quelconque des revendications précédentes, **caractérisé en ce qu'**il comprend en outre une conversion des valeurs de capacitance et de conductance du milieu en des valeurs de permittivité et de conductivité, par multiplication desdites valeurs de capacitance et de conductance par un facteur de sonde k.

31. Procédé selon la revendication 30, **caractérisé en ce qu'**il comprend en outre une détermination du facteur de sonde k, à partir d'une division de la valeur de conductivité d'une solution liquide de conductivité connue par une valeur de mesure de conductance de ladite solution.

**32.** Procédé selon l'une des revendications 30 ou 31, **caractérisé en ce qu'**il comprend en outre une détermination d'un facteur de sonde $k_a$ lié à l'aération du milieu, à une quatrième fréquence prédéterminée $f_4$.

**33.** Procédé selon la revendication 32, **caractérisé en ce que** la quatrième fréquence prédéterminée est choisie de sorte que le diélectrique est le plus stable quels que soient les changements de paramètres d'environnement.

**34.** Procédé selon l'une des revendications 32 ou 33, **caractérisé en ce que** le facteur de sonde $k_a$ représente une modification géométrique apparente du capteur lorsque des bulles sont présentes dans le milieu diélectrique.

**35.** Procédé selon l'une des revendications 32 à 34, **caractérisé en ce qu'**il comprend en outre une détermination de la capacitance Cm_cor(G, $f_4$) liée au facteur $k_a$ à la fréquence $f_4$, par comparaison de la mesure de capacitance brute au modèle de conductance Ccal(G, $f_4$).

**36.** Procédé selon la revendication 35, **caractérisé en ce que** le facteur de sonde $k_a$. est calculé en rapportant la capacitance Cm_cor(G, $f_4$) à une valeur de capacitance d'un milieu de référence non aéré.

**37.** Procédé selon l'une des revendications 32 à 36, **caractérisé en ce que** le facteur de sonde $k_a$ est utilisé pour déterminer la permittivité du milieu diélectrique corrigée des effets d'aération sur la mesure de capacitance, et pour déterminer la conductivité du milieu en corrigeant les effets de l'aération sur la mesure de conductance.

**38.** Procédé selon l'une quelconque des revendications précédentes et l'une des revendications 23 à 25 et l'une des revendications 18 à 20, **caractérisé en ce qu'**il comprend une détermination de la concentration de biomasse du milieu, en multipliant la variation de permittivité mesurée à la seconde fréquence prédéterminée f2 par un coefficient prédéterminé $\gamma$.

**39.** Procédé selon la revendication 38, **caractérisé en ce qu'**il comprend en outre une détermination du coefficient prédéterminé $\gamma$ à l'aide d'un abaque de paramètres physiques caractéristiques des cellules biologiques.

**40.** Procédé selon la revendication 38, **caractérisé en ce qu'**il comprend en outre une détermination du coefficient prédéterminé $\gamma$ à l'aide d'un étalonnage préalable dans une suspension de biomasse dont la concentration est connue.

**41.** Procédé selon l'une des revendications 32 à 40, **caractérisé en ce qu'**il comprend en outre une détermination de paramètres caractéristiques d'une dispersion diélectrique sur des milieux contenant des cellules biologiques, en utilisant au moins trois fréquences prédéterminées $f_5$, $f_6$, $f_7$.

**42.** Procédé selon la revendication 41, **caractérisé en ce qu'**il comprend en outre une détermination de capacitances liées à la dispersion Cm_cor(G, $f_5$), Cm_cor(G, $f_6$), Cm_cor(G, $f_7$), aux au moins trois fréquences prédéterminée $f_5$, $f_6$, $f_7$, en comparant des mesures de capacitance brute provenant de l'appareil aux modèles de conductance aux fréquences correspondantes Ccal(G, $f_5$), Ccal(G, $f_6$), et Ccal(G, $f_7$).

**43.** Procédé selon l'une des revendications 41 ou 42, **caractérisé en ce qu'**il comprend en outre une détermination des paramètres caractéristiques de la dispersion diélectrique, comportant :

   - une mesure d'un nombre n de valeurs mesurées de capacitance corrigée Cm_cor(G, $f_{5 à m}$) avec m = 5+n-1, à n fréquences réparties sur le domaine de fréquence correspondant à celui de la dispersion diélectrique étudiée, n étant supérieur ou égal à 3,
   - un ajustement d'une fonction multilinéaire dépendant de la fréquence et comportant n coefficients variables pour approcher au mieux les n valeurs de capacitance corrigée mesurées, et et
   - un calcul des paramètres caractéristiques de la dispersion diélectrique à partir des coefficients de la fonction multilinéaire.

**44.** Procédé selon la revendication 43, **caractérisé en ce que** la fonction multilinéaire dépendant de la fréquence est constituée par un polynôme de degré n-1.

**45.** Procédé selon la revendication 44, **caractérisée en ce qu'**il comprend un calcul d'une évaluation de la concentration de biomasse du milieu à partir des valeurs des coefficients du polynôme de degré n-1.

**46.** Procédé selon l'une des revendications 44 ou 45, **caractérisé en ce qu'**il comprend un calcul de l'évaluation de la taille des microorganismes dans le milieu à partir des coefficients du polynôme de degré n-1.

**47.** Dispositif pour évaluer une biomasse dans un milieu diélectrique à mesurer, notamment des cellules en suspension dans un fluide, comprenant des électrodes en contact avec le milieu et des moyens électroniques de traitement des signaux de capacitance et de conductance prélevés à partir desdites électrodes, ce signal de capacitance étant entaché d'erreurs de capacitance dues à une polarisation aléatoire, d'erreurs de capacitance dues à une polarisation systématique, et d'erreurs de capacitances dues aux erreurs globales provenant des moyens électroniques, les moyens électroniques de traitement étant aptes à :

- corriger les erreurs de capacitance dues à la polarisation aléatoire séparément de celles dues à la polarisation systématique et de celles dues à électronique,
- mettre en oeuvre (i) une modélisation globale de la polarisation systématique et des erreurs de capacitance dues aux erreurs globales de l'électronique sous la forme d'une équation commune du modèle d'erreur de capacitance ($C_{cal}(G, f)$) fonction de la conductance (G) du milieu et de la fréquence d'excitation (f) des électrodes conductrices, et (ii) une détermination de valeurs de capacitance corrigées (Cm_cor(G, f)) en comparant chaque mesure de capacitance brute (Cm(G, f)) provenant de l'appareil et effectuée à une fréquence prédéterminée (f) à la valeur de ladite équation commune du modèle d'erreur de capacitance ($C_{cal}(G, f)$) à ladite fréquence prédéterminée (f),
- obtenir une évaluation de la biomasse (X) à partir d'une différence ($\Delta C_x(G,f_2)$) entre un premier signal de capacitance du milieu ($C_{X\_FG\_C}(G,f_2)$) obtenu à partir d'une première mesure de capacitance brute (Cm_(G, $f_2$)) dudit milieu mesurée à une première fréquence ($f_2$), et un second signal de capacitance corrigée (Cm_cor(G,$f_3$)) obtenu à partir d'une seconde mesure de capacitance brute (Cm_(G, $f_3$)) dudit milieu mesurée à une seconde fréquence ($f_3$),

en mettant en oeuvre une correction séparée de chacun des signaux de capacitance brute (Cm_(G, $f_2$), (Cm_(G, $f_3$)) selon au moins un premier niveau de correction, ce premier niveau de correction comprenant une correction desdits signaux de capacitance brute selon ledit modèle d'erreur de capacitance ($C_{cal}(G, f)$) dépendant de la conductance (G) du milieu auxdites première et seconde fréquences, de telle sorte à obtenir respectivement des premiers et des seconds signaux de capacitance corrigés (Cm_cor(G,$f_2$), Cm_cor(G,$f_3$)).

**48.** Dispositif selon la revendication 47, **caractérisé en ce qu'**il comprend en outre des moyens pour déterminer l'erreur de capacitance due à la polarisation aléatoire à une première fréquence prédéterminée $f_1$ choisie la plus basse possible de sorte que la détermination de l'erreur de capacitance de polarisation aléatoire est peu influencée par la capacitance d'un milieu biologique.

**49.** Dispositif selon la revendication 48, **caractérisé en ce qu'**il comprend en outre des moyens pour déterminer la capacitance du milieu diélectrique à une deuxième fréquence prédéterminée $f_2$ choisie proche de la fréquence caractéristique fc du milieu, caractéristique de la dispersion $\beta$ des cellules en suspension.

**50.** Dispositif selon la revendication 49, **caractérisé en ce que** les moyens pour déterminer la capacitance du milieu diélectrique implémentent un modèle de correction résultant d'une combinaison :

- d'une mesure de capacitance de la polarisation aléatoire Cm_cor(G, $f_1$), estimée à la première fréquence prédéterminée $f_1$,
- d'une mesure de capacitance du milieu Cm_cor(G, $f_2$), estimée à la deuxième fréquence prédéterminée $f_2$,
- et d'un modèle de comportement de la polarisation aléatoire $a_{alea} \cdot f^p \cdot G^2$ dans lequel G est la conductance du milieu, $a_{alea}$ est une constante prédéfinie, et p est la pente de la polarisation.

**51.** Dispositif selon la revendication 50, mis en oeuvre pour la mesure d'un milieu contenant des cellules biologiques, **caractérisé en ce qu'**il comprend en outre des moyens pour déterminer une erreur de capacitance Cm_cor(G, $f_3$) due à la dérive thermique de l'offset de l'électronique et à des variations de la capacitance du milieu diélectrique de suspension, à une troisième fréquence prédéterminée $f_3$.

**52.** Dispositif selon la revendication 51, **caractérisé en ce qu'**il comprend en outre des moyens pour déterminer une capacitance Cm_cor(G, $f_4$) liée à un facteur de sonde apparent $k_a$ lié à l'aération du milieu, à une quatrième fréquence prédéterminée $f_4$.

**53.** Dispositif selon la revendication 52, **caractérisé en ce qu'**il comprend en outre des moyens pour déterminer des paramètres caractéristiques d'une dispersion diélectrique sur des milieux contenant des cellules biologiques, en utilisant au moins trois fréquences prédéterminées $f_5$, $f_6$, $f_7$.

**54.** Dispositif selon l'une des revendications 47 à 53, **caractérisé en ce qu'**il comprend en outre des moyens pour déterminer des paramètres caractéristiques de la dispersion diélectrique, comportant :

- des moyens pour mesurer un nombre n de valeurs de capacitance corrigée Cm_cor(G, $f_{5\ \text{à}\ m}$) avec m = 5 + n -1, à n fréquences réparties sur le domaine de fréquence correspondant à celui de la dispersion diélectrique étudiée, n étant supérieur ou égal à 3,
- des moyens pour ajuster une fonction multilinéaire dépendant de la fréquence et comportant n coefficients variables pour approcher au mieux les n valeurs de capacitance corrigée mesurées, et
- des moyens pour calculer des paramètres caractéristiques de la dispersion diélectrique à partir des coefficients de la fonction multilinéaire.

**55.** Appareil de mesure d'impédance, agencé pour fournir une mesure de la partie réelle d'une impédance et de la capacitance correspondant à cette impédance, incluant un dispositif d'évaluation de biomasse selon l'une quelconque des inventions 47 à 54.

**Patentansprüche**

**1.** Verfahren zur Evaluierung einer Biomasse in einem zu messenden dielektrischen Medium, insbesondere von Zellen in Suspension in einem Fluid, bei welchem die Messungen der Kapazität und der Leitfähigkeit mittels eines Gerätes erfolgen, welches Elektroden in Kontakt mit dem Medium umfasst, sowie elektronische Mittel zur Verarbeitung der von den Elektroden erhaltenen Kapazitäts- und Leitfähigkeitssignale, wobei das Kapazitätssignal behaftet ist mit Kapazitätsfehlern aufgrund einer zufälligen Polarisation, mit Kapazitätsfehlern aufgrund einer systematischen Polarisation und mit Kapazitätsfehlern aufgrund von allgemeinen Fehlern der elektronischen Mittel,

- wobei die Kapazitätsfehler aufgrund der zufälligen Polarisation getrennt von denjenigen aufgrund der systematischen Polarisation und denjenigen aufgrund der Elektronik korrigiert werden,
- wobei das Verfahren ferner (i) eine allgemeine Modellierung der systematischen Polarisation und der Kapazitätsfehler aufgrund von allgemeinen Fehlern der Elektronik in Form einer allgemeinen Gleichung des Kapazitätsfehler-Modells ($C_{cal}$(G, f)), Funktion der Leitfähigkeit (G) des Mediums und der Erregungsfrequenz (f) der leitenden Elektroden umfasst, und (ii) eine Bestimmung der korrigierten Kapazitätswerte (Cm_cor(G, f)) durch Vergleich jeder von dem Gerät stammenden Messung der unbearbeiteten Kapazität (Cm(G, f)) die mit einer festgelegten Frequenz (f) durchgeführt wird, mit dem Wert der allgemeinen Gleichung des Kapazitätsfehler-Modells ($C_{cal}$(G, f)) bei der festgelegten Frequenz (f),
- wobei die Bestimmung der Biomasse (X) basierend auf einer Differenz ($\Delta$Cx(G,$f_2$)) erhalten wird zwischen

einem ersten Kapazitätssignal des Mediums ($C_{X\_FG\_C}$(G,$f_2$)), basierend auf einer ersten bei einer ersten Frequenz ($f_2$) erfolgten Messung der unbearbeiteten Kapazität (Cm_(G, $f_2$)) des Mediums und einem zweiten korrigierten Kapazitätssignal (Cm_corr(G, $f_3$)), basierend auf einer bei einer zweiten Frequenz ($f_3$) erfolgten zweiten Messung der unbearbeiteten Kapazität (Cm_(G, $f_3$)) des Mediums, wobei das Verfahren eine separate Korrektur jedes der unbearbeiteten Kapazitätssignale (Cm_(G, $f_2$), (Cm_(G, $f_3$)) gemäß wenigstens einem Korrekturniveau umfasst, wobei dieses erste Korrekturniveau eine Korrektur der unbearbeiteten Kapazitätssignale gemäß dem Kapazitätsfehler-Modell ($C_{cal}$(G, f)) umfasst, abhängig von der Leitfähigkeit (G) des Mediums bei der ersten und zweiten Frequenz, so dass jeweils korrigierte erste und zweite Kapazitätssignale (Cm_corr(G,$f_2$), (Cm_corr(G,$f_3$)) erhalten werden.

**2.** Verfahren nach Anspruch 1, **dadurch gekennzeichnet, dass** die Evaluierung der Biomasse eine Evaluierung einer Konzentration der Biomasse in dem Medium umfasst.

**3.** Verfahren nach Anspruch 1 oder 2, **dadurch gekennzeichnet, dass** die Evaluierung der Biomasse eine Evaluierung einer Größe der Mikroorganismen in dem Medium umfasst.

**4.** Verfahren nach einem der vorhergehenden Ansprüche, **dadurch gekennzeichnet, dass** die Evaluierung der Biomasse eine Evaluierung eines Volumenanteils der Biomasse in dem Medium umfasst.

**5.** Verfahren nach einem der vorhergehenden Ansprüche, **dadurch gekennzeichnet, dass** es ferner ein zweites Korrekturniveau umfasst, welches eine Korrektur des ersten und zweiten korrigierten Kapazitätssignals basierend auf einer dritten, bei einer dritten Frequenz ($f_1$) durchgeführten Kapazitätsmessung ($Cm\_(G, f_1)$) umfasst, wiederum korrigiert durch eine bei der dritten Frequenz ($C_{cal}(G, f_1)$) durchgeführten Leitfähigkeitsmessung, durchgeführt.

**6.** Verfahren nach Anspruch 5, durchgeführt für ein Medium, welches Zellen in Suspension in einem Medium umfasst, **dadurch gekennzeichnet, dass** es ferner ein drittes Korrekturniveau aufweist, welches ein Verhaltensmodell für die ß-Dispersion in dem Medium verwendet.

**7.** Verfahren nach einem der vorhergehenden Ansprüche, **dadurch gekennzeichnet, dass** die allgemeine Modellgleichung $C_{cal}(G, f)$ eine polynome Form aufweist oder durch eine polynome Form in Näherung ermittelt werden kann.

**8.** Verfahren nach einem der vorhergehenden Ansprüche, **dadurch gekennzeichnet, dass** die allgemeine Modellgleichung $C_{cal}(G, f)$ mit polynomer Form von 3. oder 4. Ordnung ist.

**9.** Verfahren nach einem der vorhergehenden Ansprüche, **dadurch gekennzeichnet, dass** die allgemeine Modellgleichung $C_{cal}(G, f)$ Koeffizienten umfasst, die für eine Mehrzahl festgelegter, von dem Gerät verwendeter Frequenzen berechnet wird.

**10.** Verfahren nach einem der vorhergehenden Ansprüche, **dadurch gekennzeichnet, dass** die durch die allgemeine Modellgleichung $C_{cal}(G, f)$ korrigierten Kapazitätsfehler kapazitive Fehler als Funktion von Leitfähigkeit und Frequenz umfassen.

**11.** Verfahren nach einem der vorhergehenden Ansprüche, **dadurch gekennzeichnet, dass** die durch die allgemeine Modellgleichung $C_{cal}(G, f)$ korrigierten Kapazitätsfehler Fehler aufgrund von Leitungseffekten umfassen.

**12.** Verfahren nach einem der vorhergehenden Ansprüche, **dadurch gekennzeichnet, dass** die durch die allgemeine Modellgleichung $C_{cal}(G, f)$ korrigierten Kapazitätsfehler Fehler aufgrund von Sensorunzulänglichkeiten umfassen.

**13.** Verfahren nach einem der vorhergehenden Ansprüche, **dadurch gekennzeichnet, dass** die allgemeine Korrekturmodellgleichung $C_{cal}(G, f)$ Ungewissheit bezüglich des Entwicklungsgradienten der systematischen Polarisation als Funktion der Erregungsfrequenz ausschließt.

**14.** Verfahren nach einem der vorhergehenden Ansprüche, **dadurch gekennzeichnet, dass** die allgemeine Korrekturmodellgleichung $C_{cal}(G, f)$ das Produkt einer kombinierten Wirkung der Belüftung des Mediums und allgemeiner Elektronikfehler in dem Gerät ausschließt.

**15.** Verfahren nach einem der vorhergehenden Ansprüche und Anspruch 9, **dadurch gekennzeichnet, dass** die Koeffizienten des Korrekturmodells $C_{cal}(G, f)$ durch einen Kalibrierungsvorgang in einem Referenzmedium bestimmt werden, welches keine biologischen Zellen aufweist, und dessen Leitfähigkeit so modifiziert wird, dass der gesamte Leitfähigkeitsbereich der Vorrichtung abgedeckt wird.

**16.** Verfahren nach einem der vorhergehenden Ansprüche, **dadurch gekennzeichnet, dass** die Bestimmung des Kapazitätsfehlers aufgrund der zufälligen Polarisation bei einer festgelegten Frequenz $f_1$ berechnet wird, welche so niedrig wie möglich gewählt wird, sodass die Bestimmung des Kapazitätsfehlers der zufälligen Polarisation nicht sehr stark durch die Kapazität des Mediums beeinflusst wird.

**17.** Verfahren nach Anspruch 16, **dadurch gekennzeichnet, dass** die Kapazität der zufälligen Polarisation $Cm\_corr(G, f_1)$ bei der Frequenz $f_1$ durch Vergleich der von dem Gerät stammenden Messung der unbearbeiteten Kapazität mit dem Korrekturmodell $Ccal(G, f_1)$ berechnet wird.

**18.** Verfahren nach einem der Ansprüche 16 oder 17, **dadurch gekennzeichnet, dass** es ferner die Bestimmung der Kapazität des dielektrischen Mediums bei einer zweiten festgelegten Frequenz $f_2$ umfasst.

**19.** Verfahren nach Anspruch 18, welches für eine Messung an einer Suspension biologischer Zellen ausgeführt wird, **dadurch gekennzeichnet, dass** die zweite festgelegte Frequenz $f_2$ ähnlich der charakteristischen Frequenz fc der Suspension gewählt wird, die charakteristisch für die ß-Dispersion der Zellen in der Suspension ist.

**20.** Verfahren nach einem der Ansprüche 18 oder 19, **dadurch gekennzeichnet, dass** die Kapazität des dielektrischen Mediums Cm_corr(G, $f_2$) bei der Frequenz $f_2$ durch Vergleich der von dem Gerät stammenden Messung der unbearbeiteten Kapazität mit dem Leitfähigkeitsmodell Ccal(G, $f_2$) berechnet wird.

**21.** Verfahren nach einem der vorhergehenden Ansprüche, **dadurch gekennzeichnet, dass** es ferner eine Modellbildung der zufälligen Polarisation gemäß einem Verhaltensmodell, wie zum Beispiel $a_{alea}.f^P.G^2$ umfasst, wobei:

- G die Leitfähigkeit des Mediums ist,
- $a_{alea}$ eine festgelegte Konstante ist,
- p der Polarisierungsgradient ist.

**22.** Verfahren nach den Ansprüchen 16, 18 und 21, **dadurch gekennzeichnet, dass** die Bestimmung der Kapazität des dielektrischen Mediums ein Korrekturmodell implementiert, welches resultiert aus einer Kombination:

- der Messung der Kapazität der zufälligen Polarisation Cm_corr(G, $f_1$), geschätzt bei der ersten festgelegten Frequenz $f_1$,
- der Messung der Kapazität des Mediums Cm_corr(G, $f_2$), geschätzt bei der zweiten festgelegten Frequenz $f_2$,,
- und des Verhaltensmodells für die zufällige Polarisation $a_{alea}.f^P.G^2$.

**23.** Verfahren nach Anspruch 22, durchgeführt für die Messung von biologischen Zellsuspensionen gemäß einem Verhaltensmodells des Typs

$$\Delta\text{Kapazität } Cx(G, f) = \Delta C_{cell} \times 1/(1 + (f/f_c)^2$$

wobei $f_c$ die charakteristische Frequenz des Mediums ist.

**24.** Verfahren nach Anspruch 23, **dadurch gekennzeichnet, dass** die charakteristische Frequenz $f_c$ anhand eines Berechnungsdiagramms festgelegt wird.

**25.** Verfahren nach Anspruch 23, **dadurch gekennzeichnet, dass** die charakteristische Frequenz $f_c$ linear bestimmt wird, mittels eines Verfahrens zur Bestimmung der charakteristischen Parameter der ß-Dispersion.

**26.** Verfahren nach einem der Ansprüche 23 bis 25, **dadurch gekennzeichnet, dass** die bei der ersten festgelegten Frequenz $f_1$ gemessene Dielektrik im Wesentlichen der bei der zweiten festgelegten Frequenz $f_2$ gemessene Dielektrik entspricht.

**27.** Verfahren nach einem der Ansprüche 23 bis 26, durchgeführt für die Messung eines Mediums, welches biologische Zellen umfasst, **dadurch gekennzeichnet, dass** das Verfahren ferner eine Bestimmung eines Kapazitätsfehlers Cm_corr(G, $f_3$) aufgrund der Thermodrift des Elektronik-Offsets und aufgrund von Variationen der Kapazität des dielektrischen Suspensionsmediums, bei einer dritten festgelegten Frequenz $f_3$ umfasst.

**28.** Verfahren nach Anspruch 27, **dadurch gekennzeichnet, dass** der Kapazitätsfehler Cm_corr(G, $f_3$) bei der dritten festgelegten Frequenz $f_3$ durch Vergleich der Messung der von dem Gerät stammenden Messung der unbearbeiteten Kapazität mit dem Korrekturmodell Ccal(G, $f_3$) berechnet wird.

**29.** Verfahren nach Anspruch 28, **dadurch gekennzeichnet, dass** es ferner eine Korrektur der unbearbeiteten oder korrigierten Kapazitätsmessungen umfasst, die mit Fehlern aufgrund der Thermodrift des Elektronik-Offsets und aufgrund von Variationen der Kapazität des Suspensionsmediums behaftet sind, durch Abzug des Kapazitätsfehlers Cm_corr(G, $f_3$) von diesen Messungen.

**30.** Verfahren nach einem der vorhergehenden Ansprüche, **dadurch gekennzeichnet, dass** es ferner eine Konvertierung der Kapazitäts- und Leitwerte des Mediums in Dielektrizitätskonstanten und Leitfähigkeitswerte durch Multiplikation dieser Dielektrizitätskonstanten und Leitwerte mit einem Sensorfaktor k umfasst.

**31.** Verfahren nach Anspruch 30, **dadurch gekennzeichnet, dass** es ferner eine Bestimmung des Sensorfaktors k durch Teilen des Leitfähigkeitswertes einer flüssigen Lösung mit bekannter Leitfähigkeit durch einen gemessenen Leitwert der Lösung umfasst.

**32.** Verfahren nach einem der Ansprüche 30 oder 31, **dadurch gekennzeichnet, dass** es ferner eine Bestimmung eines Sensorfaktors $k_a$ in Verbindung mit der Belüftung des Mediums bei einer vierten festgelegten Frequenz $f_4$ umfasst.

**33.** Verfahren nach Anspruch 32, **dadurch gekennzeichnet, dass** die vierte festgelegte Frequenz so gewählt wird, dass die Dielektrik, unabhängig von den Veränderungen der Umgebungsparameter möglichst stabil ist.

**34.** Verfahren nach einem der Ansprüche 32 oder 33, **dadurch gekennzeichnet, dass** der Sensorfaktor $k_a$ für eine offensichtliche geometrische Modifikation des Sensors steht, wenn im dielektrischen Medium Blasen vorliegen.

**35.** Verfahren nach einem der Ansprüche 32 bis 34, **dadurch gekennzeichnet, dass** es ferner eine Bestimmung der Kapazität $Cm\_corr(G, f_4)$ in Verbindung mit dem Faktor $k_a$ bei der Frequenz $f_4$ durch Vergleich der Messung der unbearbeiteten Kapazität mit dem Leitfähigkeitsmodell $Ccal(G, f_3)$ umfasst.

**36.** Verfahren nach Anspruch 35, **dadurch gekennzeichnet, dass** der Sensorfaktor $k_a$ dadurch berechnet wird, dass die Kapazität $Cm\_corr(G, f_4)$ in Beziehung zu einem Kapazitätswert eines nicht belüfteten Referenzmediums gesetzt wird.

**37.** Verfahren nach einem der Ansprüche 32 bis 36, **dadurch gekennzeichnet, dass** der Sensorfaktor $k_a$ verwendet wird, um die korrigierte Dielektrizitätskonstante des dielektrischen Mediums aus den Auswirkungen der Belüftung auf die Messung der Kapazität zu bestimmen, und um die Leitfähigkeit des Mediums unter Korrektur der Auswirkungen der Belüftung auf die Messung des Leitwertes zu bestimmen.

**38.** Verfahren nach einem der vorhergehenden Ansprüche und nach einem der Ansprüche 23 bis 25 und einem der Ansprüche 18 bis 20, **dadurch gekennzeichnet, dass** es eine Bestimmung der Konzentration der Biomasse des Mediums durch Multiplizieren der bei der zweiten festgelegten Frequenz f2 gemessenen Variation der Dielektrizitätskonstante mit einem festgelegten Koeffizienten $\gamma$ umfasst.

**39.** Verfahren nach Anspruch 38, **dadurch gekennzeichnet, dass** es ferner eine Bestimmung des festgelegten Koeffizienten $\gamma$ unter Verwendung eines Berechnungsdiagramms mit für die biologischen Zellen charakteristischen physikalischen Parametern umfasst.

**40.** Verfahren nach Anspruch 38, **dadurch gekennzeichnet, dass** es ferner eine Bestimmung des festgelegten Koeffizienten $\gamma$ unter Verwendung eine vorhergehenden Kalibrierung in einem Biomassen-Suspensionsmedium mit bekannter Konzentration umfasst.

**41.** Verfahren nach einem der Ansprüche 32 bis 40, **dadurch gekennzeichnet, dass** es ferner eine Bestimmung von Parametern umfasst, die charakteristisch für eine dielektrische Dispersion auf Medien sind, die biologische Zellen enthalten, unter Verwendung wenigstens dreier festgelegter Frequenzen $f_5, f_6, f_7$.

**42.** Verfahren nach Anspruch 41, **dadurch gekennzeichnet, dass** es ferner eine Bestimmung von Kapazitäten in Verbindung mit der Dispersion $Cm\_cor(G, f_5)$, $Cm\_cor(G, f_6)$, $Cm\_cor(G, f_7)$ bei den wenigstens drei festgelegten Frequenzen $f_5, f_6, f_7$ durch den Vergleich der von dem Gerät stammenden Messung der unbearbeiteten Kapazität mit den Leitfähigkeitsmodellen bei entsprechenden Frequenzen $Ccal(G, f_5)$, $Ccal(G, f_6)$, und $Ccal(G, f_7)$ umfasst.

**43.** Verfahren nach einem der Ansprüche 41 oder 42, **dadurch gekennzeichnet, dass** es ferner eine Bestimmung der charakteristischen Parameter der dielektrischen Dispersion aufweist, umfassend:

- Messen einer Anzahl n korrigierter Kapazitätswerte $Cm\_cor(G, f_{5\,bis\,m})$ mit m=5+n-1, bei n Frequenzen verteilt über das Frequenzspektrum, entsprechend denen der untersuchten dielektrischen Dispersion, wobei n größer oder gleich 3 ist,
- eine Anpassung einer multilinearen Funktion abhängig von der Frequenz und umfassend n variable Koeffizienten für eine möglichst gute Annäherung der n gemessenen korrigierten Kapazitätswerte, und
- eine Berechnung der charakteristischen Parameter der dielektrischen Dispersion basierend auf den Koeffizienten der multilinearen Funktion.

**44.** Verfahren nach Anspruch 43, **dadurch gekennzeichnet, dass** die von der Frequenz abhängige multilineare Funktion von einem Polynom des Grads n-1 gebildet wird.

**45.** Verfahren nach Anspruch 44, **dadurch gekennzeichnet, dass** das Verfahren ferner eine Berechnung einer Bewertung der Konzentration der Biomasse des Mediums basierend auf den Werten der Koeffizienten des Polynoms des Grads n-1 umfasst.

**46.** Verfahren nach einem der Ansprüche 44 oder 45, **dadurch gekennzeichnet, dass** es eine Berechnung der Evaluierung der Größe der Mikroorganismen in dem Medium basierend auf den Werten der Koeffizienten des Polynoms des Grads n-1 umfasst.

**47.** Vorrichtung zur Evaluierung einer Biomasse in einem zu messenden dielektrischen Medium, insbesondere Zellen in Suspension in einem Fluid, welches Elektroden in Kontakt mit dem Medium umfasst, sowie elektronische Mittel zur Verarbeitung der von den Elektroden erhaltenen Kapazitäts- und Leitfähigkeitssignale, wobei das Kapazitätssignal mit Kapazitätsfehlern aufgrund einer zufälligen Polarisation, Kapazitätsfehlern aufgrund einer systematischen Polarisation und Kapazitätsfehlern aufgrund allgemeiner Fehler der Elektronik behaftet ist, wobei die elektronischen Verarbeitungsmittel ausgelegt sind für:

- die Korrektur von Kapazitätsfehlern aufgrund der zufälligen Polarisation separat von Fehlern aufgrund der systematischen Polarisation und Fehlern aufgrund der Elektronik,
- die Durchführung (i) einer allgemeinen Modellierung der systematischen Polarisation und der Kapazitätsfehler aufgrund von allgemeinen Fehlern der Elektronik in Form einer allgemeinen Gleichung des Kapazitätsfehler-Modells ($C_{cal}(G, f)$) als Funktion der Leitfähigkeit (G) des Mediums und der Erregungsfrequenz (f) der leitenden Elektroden, und (ii) eine Bestimmung der korrigierten Kapazitätswerte (Cm_corr(G, f)) durch Vergleich jeder von dem Gerät stammenden Messung der unbearbeiteten Kapazität (Cm(G, f)) bei einer festgelegten Frequenz (f), mit dem Wert der allgemeinen Gleichung des Kapazitätsfehler-Modells ($C_{cal}(G, f)$) bei der festgelegten Frequenz (f),
- eine Evaluierung der Biomasse (X) basierend auf einer Differenz ($\Delta C_X(G,f_2)$) zwischen einem ersten Kapazitätssignal des Mediums ($C_{X\_FG\_C}(G,f_2)$), basierend auf einer ersten Messung der unbearbeiteten Kapazität (Cm_(G, $f_2$)) des bei einer ersten Freuenz ($f_2$) gemessenen Mediums und einem zweiten korrigierten Kapazitätssignal (Cm_corr(G, $f_3$)), basierend auf einer zweiten Messung der unbearbeiteten Kapazität (Cm_(G, $f_3$)) des bei einer zweiten Frequenz ($f_3$) gemessenen Mediums,

durch Durchführen einer separaten Korrektur jedes der unbearbeiteten Kapazitätssignale (Cm(G, $f_2$), (Cm(G, $f_3$)) gemäß wenigstens einem ersten Korrekturniveau, wobei dieses erste Korrekturniveau eine Korrektur der unbearbeiteten Kapazitätssignale gemäß dem Kapazitätsfehler-Modell ($C_{cal}(G, f)$) umfasst, abhängig von der Leitfähigkeit (G) des Mediums bei der ersten und zweiten Frequenz, so dass jeweils korrigierte erste und zweite Kapazitätssignale (Cm_corr(G,$f_2$), (Cm_corr(G,$f_3$)) erhalten werden.

**48.** Vorrichtung nach Anspruch 47, **dadurch gekennzeichnet, dass** sie ferner Mittel zur Bestimmung des Kapazitätsfehlers aufgrund der zufälligen Polarisierung bei einer ersten festgelegten Frequenz $f_1$ umfasst, welche so niedrig wie möglich gewählt ist, sodass die Bestimmung des Kapazitätsfehlers aufgrund zufälliger Polarisation nicht sehr stark durch die Kapazität des Mediums beeinflusst wird.

**49.** Vorrichtung nach Anspruch 48, **dadurch gekennzeichnet, dass** sie ferner Mittel zur Bestimmung der Kapazität des dielektrischen Mediums bei einer zweiten festgelegten Frequenz $f_2$ umfasst, welche ähnlich der charakteristischen Frequenz fc des Mediums gewählt ist, die charakteristisch für die ß-Dispersion der Zellen in Suspension ist.

**50.** Vorrichtung nach Anspruch 49, **dadurch gekennzeichnet, dass** die Mittel zur Bestimmung der Kapazität des dielektrischen Mediums ein Korrekturmodell implementieren, welches resultiert aus einer Kombination:

- einer Messung der Kapazität der zufälligen Polarisation Cm_corr(G, $f_1$), geschätzt bei der ersten festgelegten Frequenz $f_1$,
- einer Messung der Kapazität des Mediums Cm_corr(G, $f_2$), geschätzt bei der zweiten festgelegten Frequenz $f_2$,
- und eines Verhaltensmodells für die zufällige Polarisation $a_{alea}.f^P.G^2$, wobei G die Leitfähigkeit des Mediums ist, $a_{alea}$ eine festgelegte Konstante ist, und p der Polarisationsgradient ist.

**51.** Vorrichtung nach Anspruch 50, welche ausgeführt ist für das Messen eines Mediums, das biologische Zellen aufweist, **dadurch gekennzeichnet, dass** es ferner Mittel zum Bestimmen eines Kapazitätsfehlers $Cm\_corr(G, f_3)$ aufweist, der aufgrund der Thermodrift des Elektronik-Offsets und aufgrund von Variationen der Kapazität des dielektrischen Suspensionsmediums, bei einer dritten festgelegten Frequenz $f_3$ auftritt.

**52.** Vorrichtung nach Anspruch 51, **dadurch gekennzeichnet, dass** sie ferner Mittel zum Bestimmen einer Kapazität $Cm\_corr(G, f_4)$ in Verbindung mit einem Sensorfaktor $k_a$ in Verbindung mit der Belüftung des Mediums bei einer vierten festgelegten Frequenz $f_4$ umfasst.

**53.** Vorrichtung nach Anspruch 52, **dadurch gekennzeichnet, dass** sie ferner Mittel zur Bestimmung der charakteristischen Parameter einer dielektrischen Dispersion auf Medien, welche biologische Zellen enthalten, unter Verwendung von wenigstens drei festgelegten Frequenzen $f_5$, $f_6$, $f_7$ umfasst.

**54.** Vorrichtung nach einem der Ansprüche 47 bis 53, **dadurch gekennzeichnet, dass** sie ferner Mittel zur Bestimmung der charakteristischen Parameter der dielektrischen Dispersion aufweist, umfassend:

- Mittel zum Messen einer Anzahl n korrigierter Kapazitätswerte $Cm\_cor(G, f_{5\ bis\ m})$ mit m=5+n-1, bei n Frequenzen, verteilt über das Frequenzspektrum entsprechend der untersuchten dielektrischen Dispersion, wobei n größer oder gleich 3 ist,
- Mittel zur Anpassung einer multilinearen Funktion abhängig von der Frequenz und umfassend n variable Koeffizienten für eine möglichst gute Annäherung an die n gemessenen korrigierten Kapazitätswerte, und
- Mittel zur Berechnung der charakteristischen Parameter der dielektrischen Dispersion basierend auf den Koeffizienten der multilinearen Funktion.

**55.** Gerät zum Messen der Impedanz, welches dazu ausgebildet ist, eine Messung des reellen Teils einer Impedanz und der dieser Impedanz entsprechenden Kapazität vorzusehen, einschließlich einer Vorrichtung zur Evaluierung der Biomasse nach einer der Erfindungen 47 bis 54.

**Claims**

**1.** Method for evaluating a biomass in a dielectric medium to be measured, in particular cells in suspension in a fluid, in which the capacitance and conductance measurements are carried out using an apparatus comprising electrodes in contact with the medium and electronic means for processing the capacitance and conductance signals obtained from said electrodes, this capacitance signal being contaminated with capacitance errors due to random polarization, capacitance errors due to systematic polarization, and capacitance errors due to the overall errors originating from the electronic means,

- wherein the capacitance errors due to random polarization are corrected separately from those due to systematic polarization and those due to the electronics,
- wherein the method also comprises (i) global modelling of the systematic polarization and of the capacitance errors due to the overall errors of the electronics, in the form of a common equation of the model of capacitance errors ($C_{cal}(G, f)$), a function of the conductance (G) of the medium and the excitation frequency (f) of the conductive electrodes, and (ii) determination of corrected capacitance values ($Cm\_corr(G, f)$) by comparing each raw capacitance measurement ($Cm(G, f)$) originating from the apparatus and carried out at a predetermined frequency (f), to the value of said common equation of the model of capacitance errors ($C_{cal}(G, f)$) at said predetermined frequency (f),
- wherein the evaluation of the biomass (X) is obtained from a difference ($\Delta C_X(G, f_2)$) between a first capacitance signal of said medium ($C_{X\_FG\_C}(G, f_2)$) obtained on the basis of a first measurement of the raw capacitance ($Cm\_(G, f_2)$) of said medium measured at a first frequency ($f_2$) and a second corrected capacitance signal ($Cm\_cor(G, f_3)$) obtained on the basis of a second measurement of the raw capacitance ($Cm\_(G, f_3)$) of said medium measured at a second frequency ($f_3$),

the method comprising a separate correction of each of the raw capacitance signals ($Cm\_(G, f_2)$, ($Cm\_(G, f_3)$) according to at least a first level of correction, this first level of correction comprising a correction of said raw capacitance signals according to said model of capacitance error ($C_{cal}(G, f)$) dependent on the conductance (G) of said medium at said first and second frequencies, so that corrected first and second capacitance signals ($Cm\_cor(G, f_2)$, ($Cm\_cor(G, f_3)$) are obtained respectively.

**2.** Method according to claim 1, **characterized in that** the evaluation of the biomass comprises an evaluation of a concentration of the biomass in said medium.

**3.** Method according to claim 1 or 2, **characterized in that** the evaluation of the biomass comprises an evaluation of the size of the microorganisms in said medium.

**4.** Method according to one of the preceding claims, **characterized in that** the evaluation of the biomass comprises an evaluation of a volume fraction of the biomass in said medium.

**5.** Method according to one of the preceding claims, **characterized in that** it further comprises a second level of correction comprising a correction of said first and second corrected capacitance signals, on the basis of a third measurement of capacitance ($Cm\_(G, f_1)$) carried out at a third frequency ($f_1$), itself corrected by a conductance measurement carried out at the third frequency ($Ccal(G, f_1)$).

**6.** The method according to claim 5, carried out for a medium comprising cells in suspension in a medium, **characterized in that** it further comprises a third level of correction using a model of the behavior of the ß-dispersion in said medium.

**7.** The method according to one of the preceding claims, **characterized in that** the common equation of the model $C_{cal}(G, f)$ is of polynomial form, or can be approximated by a polynomial form.

**8.** The method according to one of the preceding claims, **characterized in that** the common equation of the model $C_{cal}(G, f)$ of polynomial form is of order 3 or 4.

**9.** The method according to one of the preceding claims, **characterized in that** the common equation of the model $C_{cal}(G, f)$ has coefficients calculated for a plurality of predetermined frequencies used by the apparatus.

**10.** The method according to one of the preceding claims, **characterized in that** the capacitance errors corrected by the common equation of the model $C_{cal}(G, f)$ comprise capacitive errors as a function of conductance and frequency.

**11.** The method according to one of the preceding claims, **characterized in that** the capacitance errors corrected by the common equation of the model $C_{cal}(G, f)$ comprise errors linked to line effects.

**12.** The method according to one of the preceding claims, **characterized in that** the capacitance errors corrected by the common equation of the model $C_{cal}(G, f)$ comprise errors linked to imperfections in the sensor.

**13.** The method according to one of the preceding claims, **characterized in that** the correction model of common equation $C_{cal}(G, f)$ eliminates uncertainty regarding the systematic polarization development gradient as a function of the excitation frequency.

**14.** The method according to one of the preceding claims, **characterized in that** the correction model of common equation $C_{cal}(G, f)$ eliminates the product of a combined effect of aeration of the medium and overall errors of electronics within the apparatus.

**15.** The method according to one of the preceding claims and to claim 9, **characterized in that** the coefficients of the correction model $C_{cal}(G, f)$ are determined from a calibration operation in a reference medium containing no biological cells and the conductance of which is modified so as to cover the full scale of the conductance range of the device.

**16.** The method according to one of the preceding claims, **characterized in that** the capacitance error due to random polarization is calculated at a predetermined frequency $f_1$ chosen to be as low as possible so that the determination of the capacitance error due to random polarization is not very much influenced by the capacitance of the medium.

**17.** The method according to claim 16, **characterized in that** the random polarization capacitance $Cm\_cor(G, f_1)$ is calculated at the frequency $f_1$ by comparison between the raw capacitance measurement originating from the apparatus and the correction model $Ccal(G, f_1)$.

**18.** The method according to one of claims 16 or 17, **characterized in that** it further comprises determining the capacitance of the dielectric medium at a second predetermined frequency $f_2$.

**19.** The method according to claim 18, carried out for a measurement carried out on a biological cell suspension, **characterized in that** the second predetermined frequency $f_2$ is chosen close to the characteristic frequency fc of said suspension, characteristic of the ß-dispersion of the cells in suspension.

**20.** The method according to one of claims 18 or 19, **characterized in that** the capacitance of the dielectric medium $Cm\_cor(G, f_2)$ is calculated at the frequency $f_2$, by comparison between the raw capacitance measurement originating from the apparatus and the conductance model $Ccal(G, f_2)$.

**21.** The method according to one of the preceding claims, **characterized in that** it further comprises a modelling of the random polarization according to a behavior model such as $a_{alea}.f^P.G^2$, in which:

- G is the conductance of the medium,
- $a_{alea}$ is a predefined constant,
- p is the polarization gradient.

**22.** The method according to claims 16, 18 and 21, **characterized in that** the determination of the capacitance of the dielectric medium implements a correction model resulting from a combination:

- of the random polarization capacitance measurement $Cm\_cor(G, f_1)$ estimated at the first predetermined frequency $f_1$,
- of the capacitance measurement of the medium $Cm\_cor(G, f_2)$, estimated at the second predetermined frequency $f_2$,
- and of the behaviour model of the random polarization $a_{alea}.f^P.G^2$.

**23.** The method according to claim 22, carried out for the measurement of biological cell suspensions according to a behaviour model of the type

$$\Delta capacitance \ Cx(G, f) = \Delta C_{cell} \ x1/(1 + (f/f_c)^2)$$

where $f_c$ is the characteristic frequency of the medium.

**24.** The method according to claim 23, **characterized in that** the characteristic frequency $f_c$ is predetermined from a calculation chart.

**25.** The method according to claim 23, **characterized in that** the characteristic frequency $f_c$ is determined in line by a method for the determination of characteristic parameters of a ß-dispersion.

**26.** The method according to one of claims 23 to 25, **characterized in that** the dielectric measured at the first predetermined frequency $f_1$ is substantially identical to the dielectric measured at the second predetermined frequency $f_2$.

**27.** The method according to one of claims 23 to 26, implemented for the measurement of a medium containing biological cells, **characterized in that** the method further comprises determining a capacitance error $Cm\_cor(G, f_3)$ due to thermal drift of an offset of the electronics and to variations in the capacitance of the dielectric suspension medium, at a third predetermined frequency $f_3$.

**28.** The method according to claim 27, **characterized in that** the capacitance error $Cm\_cor(G, f_3)$ is calculated at the third predetermined frequency $f_3$ by comparison between the raw capacitance measurement originating from the apparatus and the correction model $Ccal(G, f_3)$.

**29.** The method according to claim 28, **characterized in that** the method further comprises correcting raw or corrected capacitance measurements, contaminated with errors due to the thermal drift of the offset of the electronics and to variations in capacitance of the suspension medium, by subtracting from these measurements the capacitance error $Cm\_cor(G, f_3)$.

**30.** The method according to one of the preceding claims, **characterized in that** it further comprises converting the capacitance and conductance values of the medium to permittivity and conductivity values, by multiplication of said capacitance and conductance values by a probe factor k.

31. The method according to claim 30, **characterized in that** it further comprises determining the probe factor k, from division of the conductivity value of a liquid solution of known conductivity by a conductance measurement value of said solution.

32. The method according to claim 30 or claim 31, **characterized in that** it further comprises determining a probe factor $k_a$ linked to the aeration of the medium, at a fourth predetermined frequency $f_4$.

33. The method according to claim 32, **characterized in that** the fourth predetermined frequency is chosen such that the dielectric is the most stable whatever the changes in environmental parameters.

34. The method according to one of claims 32 or 33, **characterized in that** the probe factor $k_a$ represents an apparent geometric modification of the sensor when bubbles are present in the dielectric medium.

35. The method according to one of claims 32 to 34, **characterized in that** it further comprises determining the capacitance $Cm\_cor(G, f_4)$ linked to the factor $k_a$ at the frequency $f_4$ by comparison of the raw capacitance measurement with the conductance model $Ccal(G, f_4)$.

36. The method according to claim 35, **characterized in that** the probe factor $k_a$ is calculated by relating the capacitance $Cm\_cor(G, f_4)$ to a capacitance value of a non-aerated reference medium.

37. The method according to one of claims 32 to 36, **characterized in that** the probe factor $k_a$ is used in order to determine the corrected permittivity of the dielectric medium from the effects of aeration on the capacitance measurement, and in order to determine the conductivity of the medium by correcting the effects of aeration on the conductance measurement.

38. The method according to one of the preceding claims and one of claims 23 to 25 and one of claims 18 to 20, **characterized in that** it comprises determining the concentration of biomass of the medium, by multiplying the permittivity variation measured at the second predetermined frequency f2 by a predetermined coefficient $\gamma$.

39. The method according to claim 38, **characterized in that** it further comprises determining the predetermined coefficient $\gamma$ using a calculation chart of physical parameters characteristic of biological cells.

40. The method according to claim 38, **characterized in that** it further comprises determining the predetermined coefficient $\gamma$ using a previous calibration in a biomass suspension the concentration of which is known.

41. The method according to one of claims 32 to 40, **characterized in that** it further comprises a determination of parameters characteristic of a dielectric dispersion on media containing biological cells, by using at least three predetermined frequencies $f_5$, $f_6$, $f_7$.

42. The method according to claim 41, **characterized in that** it further comprises determining capacitances linked to the dispersion $Cm\_cor(G, f_5)$, $Cm\_cor(G, f_6)$, $Cm\_cor(G, f_7)$ at the at least three predetermined frequencies $f_5$, $f_6$, $f_7$ by comparing raw capacitance measurements originating from the apparatus to the conductance models at the corresponding frequencies $Ccal(G, f_5)$, $Ccal(G, f_6)$, and $Ccal(G, f_7)$.

43. The method according to one of claims 41 or 42, **characterized in that** it further comprises determining the characteristic parameters of the dielectric dispersion, comprising:

- measuring a number n of corrected measured capacitance values $Cm\_cor(G, f_{5\ to\ m})$ with m=5+n-1, at n frequencies distributed over the frequency range corresponding to that of the dielectric dispersion studied, n being greater than or equal to 3,
- an adjustment of the multilinear function depending on the frequency and comprising n variable coefficients in order best to approach the n values of measured corrected capacitance, and
- calculating the parameters characteristic of the dielectric dispersion from the coefficients of the multilinear function.

44. The method according to claim 43, **characterized in that** the multilinear function dependent on the frequency is constituted by a polynomial of degree n-1.

45. The method according to claim 44, **characterized in that** the method further comprises calculating an evaluation of the concentration of biomass of the medium from the values of the coefficients of the polynomial of degree n-1.

46. The method according to one of claims 44 or 45, **characterized in that** it comprises calculating the evaluation of the size of the microorganisms in the medium from the coefficients of the polynomial of degree n-1.

47. Device for evaluating a biomass in a dielectric medium to be measured, in particular cells in suspension in a fluid, comprising electrodes in contact with the medium and electronic means for processing capacitance and conductance signals taken from said electrodes, the capacitance signal being contaminated with capacitance errors due to random polarization, capacitance errors due to systematic polarization and capacitance errors due to global errors from electronic means, the electronic processing means being adapted for:

- correcting capacitance errors due to random polarization separately from errors due to systematic polarization and errors due to electronics,
- carrying out (i) a global modelling of systematic polarization and capacitance errors including errors due to global errors of electronics in the form of a common equation ($C_{cal}(G, f)$), a function of the conductance (G) of the medium and excitation frequency (f) of the conductive electrodes, and (ii) for determining corrected capacitance values ($Cm\_cor(G, f)$), by comparing each raw capacitance measurement ($Cm(G, f)$) originating from the apparatus and carried out at a predetermined frequency (f), with the value of said common equation of the model of capacitance errors ($C_{cal}(G, f)$) at said predetermined frequency (f),
- achieving an evaluation of the biomass (X) based on a difference ($\Delta C_X(G,f_2)$) between a first capacitance signal of said medium ($C_{X\_FG\_C}(G,f_2)$) obtained on the basis of a first measurement of the raw capacitance ($Cm(G, f_2)$) of said medium measured at a first frequency ($f_2$) and a second corrected capacitance signal ($Cm\_cor(G, f_3)$) obtained on the basis of a second measurement of the raw capacitance ($Cm(G, f_3)$) of said medium measured at a second frequency ($f_3$), by carrying out a separate correction of each of the raw capacitance signals ($Cm\_(G, f_2)$, ($Cm\_(G, f_3)$) according to at least a first level of correction, this first level of correction comprising a correction of said raw capacitance signals according to said model of capacitance error ($C_{cal}(G, f)$) dependent on the conductance (G) of said medium at said first and second frequencies, so that corrected first and second capacitance signals ($Cm\_cor(G,f_2)$, ($Cm\_cor(G,f_3)$) are obtained respectively.

48. Device according to claim 47, **characterized in that** it further comprises means for determining the capacitance error due to random polarization at a first predetermined frequency $f_1$ chosen to be as low as possible, such that determination of the random polarization capacitance error is only slightly influenced by the capacitance of a biological medium.

49. Device according to claim 48, **characterized in that** it further comprises means for determining the capacitance of the dielectric medium at a second predetermined frequency $f_2$ chosen to be close to the characteristic frequency fc of the medium, characteristic of the ß-dispersion of the cells in suspension.

50. Device according to claim 49, **characterized in that** the means for determining the capacitance of the dielectric medium implement a correction model resulting from a combination:

- of a capacitance measurement of random polarization $Cm\_cor(G, f_1)$, estimated at the first predetermined frequency $f_1$,
- of a capacitance measurement of the medium $Cm\_cor(G, f_2)$, estimated at the second predetermined frequency $f_2$,
- and of a behaviour model of random polarization $a_{alea}.f^p.G^2$, in which G is the conductance of the medium, $a_{alea}$ is a predefined constant and p is the polarization gradient.

51. Device according to claim 50, carried out for measuring a medium containing biological cells, **characterized in that** it also comprises means for determining a capacitance error $Cm\_cor(G, f_3)$ due to the thermal drift of the offset of the electronics and to variations in the capacitance of the dielectric suspension medium, at a third predetermined frequency $f_3$.

52. Device according to claim 51, **characterized in that** it further comprises means for determining a capacitance $Cm\_cor(G, f_4)$ linked to a probe factor $k_a$ linked to the aeration of the medium, at a fourth predetermined frequency $f_4$.

**53.** Device according to claim 52, **characterized in that** it further comprises means for determining parameters characteristic of a dielectric dispersion on media containing biological cells, by using at least three predetermined frequencies $f_5$, $f_6$, $f_7$.

**54.** Device according to claims 47 to 53, **characterized in that** it also comprises means for determining parameters characteristic of the dielectric dispersion, comprising:

- means for measuring a number n of corrected capacitance values $Cm\_cor(G, f_{5 \text{ to } m})$ with m=5+n-1, at n frequencies distributed over the frequency range corresponding to that of the dielectric dispersion studied, n being greater than or equal to 3,
- means for adjusting a multilinear function dependent on frequency and comprising n variable coefficients for best approaching the n measured corrected capacitance values, and
- means for calculating parameters characteristic of the dielectric dispersion from the coefficients of the multilinear function.

**55.** An impedance measurement apparatus, arranged for providing a measurement of the real part of an impedance and of the capacitance corresponding to this impedance, including a device for evaluating a biomass according to any one of the inventions 47 to 54.

Modèle de comportement de cellule biologique : dispersion β

FIG.1

FIG.2 (art antérieur)

Polarisation mesurée sans ligne : C(f)

FIG.3

Erreurs de l'électronique mesurées sur étalons résistifs

FIG.4

Erreur capteur : C(G)

## FIG.5

Effets de ligne mesurés sur étalons résistifs : C(G)

## FIG.6

effet des polarisations systématique et aléatoire : C(f)

FIG.7

FIG.8

FIG.9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0282532 A
- EP 0281602 A
- EP 0620919 A
- EP 1138758 A
- EP 1085316 A
- FR 2812725
- FR 2835921

**Littérature non-brevet citée dans la description**

- **FRICKE.** Relation of the permittivity of biological cell suspensions to fractional cell volume. *Nature,* 1953, vol. 172 (4381), 731-732
- **SCHWANN.** Electrical properties of tissues and cell suspensions. *Adv. Biol. Med. Phys.,* 1957, vol. 5, 147-209
- **SIUGURA et al.** Dielectric behavior of yeast cells in suspension. *J.Gen.App.Microbiol.,* 1964, vol. 10 (2), 163-174
- Determination of biomass concentration by capacitance measurement. *Biotechnol. Bioeng.,* 1979, vol. 21 (6), 1097-1103
- *Hewlett-Packard Journal: technical information from the laboratories of Hewlett-Packard Company,* Janvier 1980, vol. 31 (1), 22-31
- *Hewlett-Packard Journal: technical information from the laboratories of Hewlett-Packard Company,* Septembre 1981, vol. 32 (9), 22-28
- **CLARKE et al.** Sensors for bioreactor monitoring and control - a perspective. *J. Biotechnol,* vol. 1, 135-158
- **YARDLEY, KELL et al.** On-line, real-time measurements of cellular biomass using dielectric spectroscopy. *Biotechnology & Genetic Engineering Reviews,* 2000, vol. 17, 3-35
- **YARDLEY et al.** *Bioelectrochemistry,* 2000, vol. 51, 53-65
- Determination of biological impedances. **SCHWAN.** Physical techniques in Biological research. Academic Press, 1963, vol. 6, 323-407
- **AINSI, SUGIURA et al.** Dielectric behavior of yeast cells in suspension. *J.Gen.App.Microbiol.,* 1964, vol. 10 (2), 163-174